# EUROPEAN PATENT SPECIFICATION

(11) **EP 4 437 952 B1**
(45) Date of publication and mention of the grant of the patent: **02.12.2026**
(21) Application number: 22897008.3
(22) Date of filing: 18.03.2022
(51) Int. Cl.: A61B 5/145, A61B 5/00, A61B 17/34

(54) **SENSOR APPLICATOR FOR PERCUTANEOUS IMPLANTATION OF SENSOR, AND MEDICAL DEVICE**
SENSORAPPLIKATOR ZUR PERKUTANEN IMPLANTATION EINES SENSORS UND MEDIZINISCHE VORRICHTUNG
APPLICATEUR DE CAPTEUR POUR IMPLANTATION PERCUTANÉE D'UN CAPTEUR, ET DISPOSITIF MÉDICAL

(30) Priority: 26.11.2021 CN 202111417361; 26.11.2021 CN 202210216651
(43) Date of publication of application: 02.10.2024
(73) Proprietor: Shanghai Microport Lifesciences Co., Ltd., Shanghai 201318 (CN)
(72) Inventor: LIU, Wenjie, Shanghai 201318 (CN); CHEN, Xun, Shanghai 201318 (CN); HAO, Xuan, Shanghai 201318 (CN); FU, Xiangyang, Shanghai 201318 (CN); QIU, Dan, Shanghai 201318 (CN)
(74) Representative: Patentship Patentanwaltsgesellschaft mbH
(86) International application number: PCT/CN2022/081737
(87) International publication number: WO 2023/092907

(56) References cited:
- WO-A1-2020/231405
- WO-A1-2021/088544
- CN-A- 110 720 930
- CN-A- 112 006 653
- CN-A- 112 294 300
- CN-A- 112 494 038
- CN-A- 113 082 362
- CN-A- 113 827 231
- CN-U- 212 015 601
- US-A1- 2021 030 326

## Description

### TECHNICAL FIELD

The present application relates to the technical field of medical instruments and, more specifically, to a sensor applicator for percutaneous implantation of a sensor, and a medical device.

### BACKGROUND

For a target subject suffering from diabetes, measurement of blood glucose concentration is an essential daily task. Currently, a common measurement method on the market is to collect blood from the fingertips and use a finger blood glucose concentration meter to measure the blood glucose concentration. However, this measurement method has great limitations because it can only measure the blood sugar concentration value of the target subject at a single point in time and cannot continuously monitor the blood glucose concentration level. To address this shortcoming, CGM (continuous glucose monitoring) came into being.

The principle of CGM is to monitor the glucose concentration in the interstitial fluid of the subcutaneous tissue through a glucose sensor. It is a monitoring technology that indirectly reflects the blood glucose concentration level. It can continuously provide blood glucose concentration information throughout the day, enable insights into the trend of blood glucose concentration fluctuations, and provide doctors with very effective judgment basis for diagnosis and treatment of target subjects.

Existing CGM products are complex in structure, high in production costs, and cumbersome for users to use, resulting in a poor user experience. A known devices is disclosed in WO 2021/088544 A1.

### SUMMARY OF THE INVENTION

An object of the present application is to provide a sensor applicator for percutaneous implantation of a sensor, and a medical device, which can solve the problems of complex structure and high cost of the existing applicator.

In order to solve the above technical problems, the present application provides an applicator for percutaneous implantation of sensor, comprising a first housing and a needle insertion assembly, wherein the needle insertion assembly is arranged inside the first housing and is configured to carry a sensor assembly comprising a sensor; wherein the needle insertion assembly comprises a needle holder and a second housing, wherein the needle holder is configured to fix an implant needle, a first mounting seat configured to releasably accommodate the needle holder is provided at a distal end of the second housing, and a proximal end of the second housing is configured to be detachably connected to the sensor assembly; wherein the first mounting seat is connected to a first elastic member arranged along an axial direction of the first housing, a distal end of the first elastic member is connected to the needle holder, and a proximal end of the first elastic member is connected to a proximal end of the first mounting seat; wherein the first mounting seat comprises a plurality of elastic claws, between which a first accommodation space configured to accommodate the needle holder and the first elastic member is formed; wherein the first housing comprises a clasping member and a pressing portion, wherein the clasping member is arranged inside the first housing along the axial direction thereof, wherein before the needle insertion assembly is moved to a position close to a proximal end of the first housing, the clasping member can be sleeved over the elastic claws to clasp the elastic claws; after the pressing portion is triggered, the needle insertion assembly is moved toward the proximal end of the first housing to percutaneously implant the sensor under a skin of a target subject until the clasping member is separated from the elastic claws, so that distal ends of the elastic claws can be separated from the needle holder, and then the first elastic member instantly causes the needle holder to automatically move toward a distal end of the first housing.

Optionally, before the needle insertion assembly is moved to the position close to the proximal end of the first housing, the first elastic member is in a compressed state so that the needle holder is accommodated in the first mounting seat; wherein after the needle insertion assembly is moved to the position close to the proximal end of the first housing, under an action of the first elastic member, the needle holder can be released from the first mounting seat and move toward the distal end of the first housing.

Optionally, the first mounting seat further comprises a base, wherein the plurality of elastic claws extend along an axial direction of the base toward the distal end of the first housing, and the first accommodation space is formed between the base and the elastic claws; wherein before the needle insertion assembly is moved to the position close to the proximal end of the first housing, the distal ends of the elastic claws abut against a distal end of the needle holder to fix the needle holder in the first accommodation space; wherein after the needle insertion assembly is moved to the position close to the proximal end of the first housing, the distal ends of the elastic claws are separated from the needle holder under the action of the first elastic member, so that the needle holder can be released from the first mounting seat and move toward the distal end of the first housing.

Optionally, a second elastic member is provided inside the first housing along an axial direction thereof, a distal end of the second elastic member is connected to the distal end of the first housing, and a proximal end of the second elastic member is connected to the second housing; wherein before the pressing portion is triggered, the second elastic member is in a compressed state; wherein after the pressing portion is triggered, under an action of the second elastic member, the second housing moves toward the position close to the proximal end of the first housing.

Optionally, a plurality of abutment members are provided at the distal end of the second housing, an abutment platform matched with the abutment members is provided on an inner wall of the first housing, and the abutment platform is close to a position where the pressing portion is located; wherein before the pressing portion is triggered, the abutment members abut against the abutment platform; wherein after the pressing portion is triggered, the abutment members can be separated from the abutment platform.

Optionally, the second housing is provided with a sliding slot extending through the proximal and distal ends of the second housing along the axial direction of the first housing, a slide rail matched with the sliding slot is provided on an inner wall of the first housing, and a stopper is provided at a proximal end of the slide rail.

Optionally, the pressing portion comprises a main body and first cantilevers arranged on both sides of the main body, wherein the first cantilevers extend outward along the main body, the first cantilevers are connected to an inner wall of the first housing, a first mounting hole corresponding to the main body is defined on the first housing, the main body is installed in the first mounting hole, the main body can move back and forth in the first mounting hole, the first cantilevers each are arranged in an arc shape, a second mounting hole is defined on a side of each of the first cantilevers away from the main body, and a mounting pillar matched with the second mounting hole is provided on the inner wall of the first housing.

Optionally, the needle holder comprises a fixing seat and a plurality of guide pillars extending along an axial direction of the fixing seat toward the proximal end of the first housing, wherein a second accommodation space configured to accommodate the first elastic member is formed between the fixing seat and the guide pillars, an opening is defined at a proximal end of the fixing seat, a connecting portion is provided inside the fixing seat along an axial direction thereof, a plurality of first clamping portions are provided at a distal end of the implant needle, and first clamping slots matched with the first clamping portions are provided on the connecting portion.

Optionally, a plurality of second cantilevers are provided at the distal end of the implant needle, the second cantilevers extend outward along an axial direction of the implant needle, and the first clamping portions are provided on distal ends of the second cantilevers.

Optionally, an anti-slip structure is provided on an outer wall of the first housing; wherein the anti-slip structure comprises one or more of ridges, bumps, recesses or textures arranged on the first housing.

In order to solve the above technical problems, the present application also provides a medical device, comprising a sensor assembly and the applicator mentioned above, wherein the sensor assembly comprises a sensor and a transmitter that are connected with each other, and when the sensor is percutaneously implanted under the skin of the target subject, the transmitter can receive a detection result from the sensor and transmit the detection result.

Optionally, the sensor assembly further comprises a foundation, wherein the sensor and the transmitter are installed on the foundation, and the proximal end of the second housing is provided with a second mounting seat configured to releasably accommodate the foundation, and the foundation is detachably connected to the second mounting seat.

Optionally, a plurality of second clamping slots are provided on an outer periphery of the foundation, and second clamping portions matched with the second clamping slots are provided on an inner wall of the second mounting seat.

Optionally, a proximal end of the foundation is provided with a contact surface extending outwardly along a circumferential direction of the foundation, the contact surface is configured to contact the skin of the target subject, and an adhesive is provided on the contact surface.

Optionally, the medical device further comprises a protective cover, wherein the sensor assembly and the needle insertion assembly are sealed in a space enclosed by the first housing and the protective cover, the protective cover is detachably installed at a proximal end of the first housing, and a release liner that cooperates with the adhesive is provided at a distal end of the protective cover

Optionally, a through hole allowing the implant needle to pass therethrough is defined on the foundation, a proximal end of the sensor is provided with a pin protruding out of the through hole, and a groove configured to wrap the pin is defined on the implant needle along an axial direction thereof.

Optionally, a through hole allowing the implant needle of the applicator to pass therethrough is defined on the sensor assembly, and an elastic contact switch is provided in the through hole, when the needle holder is accommodated in the first mounting seat, the elastic contact switch is in a non-conducting state, and when the needle holder is released from the first mounting seat and moves to a position close to the distal end of the first housing, the elastic contact switch is in a conducting state.

Optionally, the elastic contact switch comprises a pair of conductive members arranged opposite to each other, wherein at least one of the two conductive members is elastic, the implant needle comprises a needle handle and a needle body that are connected to each other, wherein the needle handle is made of insulating material, when the needle holder is accommodated in the first mounting seat, one of the conductive members abuts against one side of the needle handle, and the other one of conductive members abuts against a further side of the needle handle, so that the elastic contact switch is in a non-conducting state, and when the needle holder is released from the first mounting seat and moves to a position close to the distal end of the first housing, the two conductive members contact each other, so that the elastic contact switch is in a conducting state.

Optionally, the elastic conductive members each comprise a connecting plate, a telescopic member and a contact terminal, wherein the telescopic member is fixed to the connecting plate, and the contact terminal is connected to an end of the telescopic member away from the connecting plate, and wherein under an action of the telescopic member, the contact terminal moves toward a position away from the connecting plate until the contact terminal contacts the other one of the conductive members, so that the elastic contact switch is conducting.

Optionally, the telescopic member comprises a hollow sleeve and a third elastic member arranged in the sleeve, wherein the sleeve is fixed to the connecting plate, one end of the third elastic member is connected to the sleeve, and a further end of the third elastic member is connected to the contact terminal.

Optionally, the telescopic member and the contact terminal are structured integrally, wherein the telescopic member is an inclined spring sheet, and the contact terminal is formed by bending one end of the telescopic member away from the connecting plate.

Optionally, the medical device further comprises a sterilization box, wherein the sensor assembly and the applicator are sterilized and installed in the sterilization box.

In summary, compared with the prior art, the sensor applicator for percutaneous implantation of a sensor, and a medical device according to the present application have the following advantages. The applicator according to the present application includes a first housing and an insertion needle assembly, and the needle insertion assembly is disposed inside the first housing and is configured to carry a sensor assembly including the sensor. The needle insertion assembly includes a needle holder and a second housing. The needle holder is configured to fix the implant needle. The distal end of the second housing is provided with a first mounting seat for releasably accommodating the needle holder, and the proximal end of the second housing is configured to detachably connect with the sensor assembly. The first mounting seat is connected to a first elastic member arranged along the axial direction of the first housing. The distal end of the first elastic member is connected to the needle holder, and the proximal end of the first elastic member is connected to the proximal end of the first mounting seat. The first mounting seat includes a plurality of elastic claws, between which the first accommodation space for accommodating the needle holder and the first elastic member can be formed. The first housing includes a clasping member and a pressing portion. The clasping member is provided inside the first housing and arranged along the axial direction of the first housing. Before the needle insertion assembly is moved to a position close to the proximal end of the first housing, the clasping member can be sleeved on the elastic claws to clasp the elastic claws tightly. After the pressing portion is triggered, the needle insertion assembly is moved toward the proximal end of the first housing to percutaneously implant the sensor under the skin of the target subject until the clasping member can be separated from the elastic claws, so that the distal ends of the elastic claws can be separated from the needle holder, and then the first elastic member instantly causes the needle holder to automatically move toward the distal end of the first housing. Therefore, by triggering the pressing portion, the needle insertion assembly can be moved from a position away from the proximal end of the first housing to a position close to the proximal end of the first housing, so that the sensor assembly carrying the sensor can be transported to the skin surface of the target subject, and the sensor can be percutaneously implanted under the skin of the target subject to detect parameters such as blood glucose concentration in the blood of the target subject. The needle holder is releasably accommodated in the first mounting seat. Therefore, when the sensor is percutaneously implanted under the skin of the target subject, the clasping member can be separated from the elastic claws, so that the distal ends of the elastic claws can be separated from the needle holder, and then the needle holder can be instantly caused to automatically move toward the distal end of the first housing under the action of the resilience force of the first elastic member, so that the implant needle can instantly rebound together with the needle holder into the first housing, effectively avoiding accidental injury to the operator or other personnel by the sharp implant needle. In conclusion, the applicator according to the present application is not only simple in structure and low in cost, but also easier to operate. Since the medical device according to the present application includes the applicator described above, it has all the advantages of the medical device described above, so it will not be described in detail.

### BRIEF DESCRIPTION OF THE DRAWINGS

Fig. 1 is a schematic diagram showing an overall structure of an applicator according to an embodiment of the present application.
Fig. 2 is a cross-sectional view of the medical device before the pressing portion is triggered according to an embodiment of the present application.
Fig. 3 is a cross-sectional view of the applicator after a needle returning action is completed according to an embodiment of the present application.
Fig. 4 is a schematic diagram showing a structure of a needle insertion assembly before returning of the needle according to an embodiment of the present application.
Fig. 5 is a schematic diagram showing a three-dimensional view of the structure of the applicator after the needle returning action is completed according to an embodiment of the present application.
Fig. 6 is a schematic diagram of a first housing according to an embodiment of the present application.
Fig. 7 is a schematic diagram showing the structure of a pressing portion according to an embodiment of the present application.
Fig. 8 is a schematic diagram showing the structure of a needle holder according to an embodiment of the present application.
Fig. 9 is a schematic diagram showing the structure of an implant needle according to an embodiment of the present application.
Fig. 10 is a schematic diagram showing a three-dimensional view of the structure of a sensor assembly according to an embodiment of the present application.
Fig. 11 is a cross-sectional view of the sensor assembly according to an embodiment of the present application.
Fig. 12 is a schematic diagram showing a structure where the second housing and the sensor assembly are separated from each other according to an embodiment of the present application.
Fig. 13 is a schematic diagram showing the connection between the sensor assembly and the implant needle according to an embodiment of the present application.
Fig. 14 is a cross-sectional view of Fig. 13.
Fig. 15 is a schematic diagram of an elastic contact switch in its non-conducting state according to an embodiment of the present application.
Fig. 16 is a schematic diagram showing the structure of a conductive member which is elastic according to an embodiment of the present application.
Fig. 17 is a schematic diagram of an elastic contact switch in its conducting state according to an embodiment of the present application.
Fig. 18 is a schematic diagram of an elastic contact switch in its conducting state according to another embodiment of the present application.
Fig. 19 is a schematic diagram showing an overall structure of a medical device according to another embodiment of the present application.
Fig. 20 is a schematic diagram showing an exploded view of the structure of the medical device shown in Fig. 19.
Fig. 21 is a schematic diagram showing an overall structure of a medical device according to still another embodiment of the present application.
Fig. 22 is a schematic diagram showing a partial structure of the medical device shown in Fig. 21.

The reference numerals are as follows: first housing 100; ridge 110; clasping member 120; abutment platform 130; slide rail 140; stopper 141; first mounting hole 150; mounting pillar 160; pressing portion 200; main body 210; first cantilever 220; second mounting hole 221; rib 211; needle holder 300; implant needle 310; first clamping portion 311; second cantilever 312; groove 313; needle handle 314; needle body 315; fixing seat 320; connecting portion 321; first clamping slot 3211; guide pillar 330; second accommodation space 340; second housing 400; first mounting seat 410; base 411; elastic claw 412; first accommodation space 413; abutment member 420; inclined surface 421; sliding slot 430; second mounting seat 440; second clamping portion 441; first elastic member 500; second elastic member 600; sensor assembly 700; sensor 710; conductive element 711; pin 712; transmitter 720; foundation 730; second clamping slot 731; through hole 732; circuit board 740; contact surface 750; elastic contact switch 760; conductive member 761, 761a, 761b, 761c, 761d; connecting plate 7611, 7611b; first plate body 76111; second plate body 76112; telescopic member 7612, 7612b; sleeve 76121; third elastic member 76122; contact terminal 7613, 7613b; protective cover 800; protective seat 810; sterilization box 900, box body 910; box cover 920.

### DETAILED DESCRIPTION

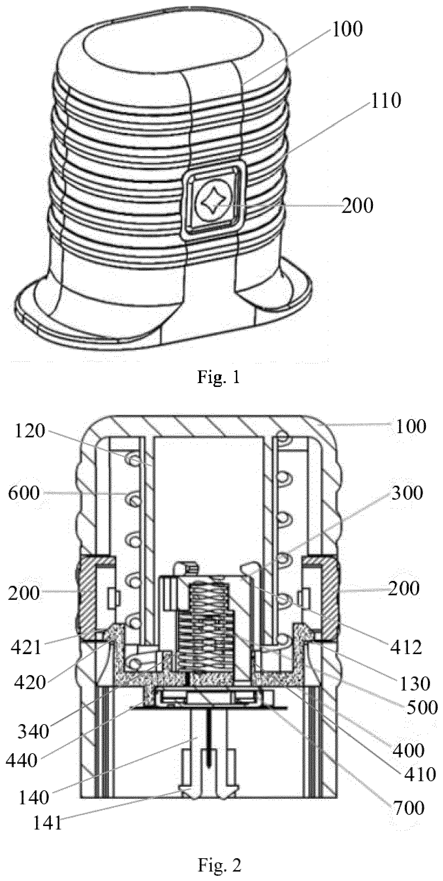

The sensor applicator for percutaneous implantation of a sensor, and medical device proposed in the present application will be described in greater detail below with reference to specific embodiments which are to be read in conjunction with the accompanying Figs. 1-22. Advantages and features of the present application will be apparent from the description and appended claims below. It is noted that the figures are provided in a very simplified form not necessarily presented to scale, with their only intention to facilitate convenience and clarity in explaining the disclosed embodiments. In order to make the objects, features and advantages of the present application more clearly understood, please refer to the accompanying drawings. It should be noted that the structures, proportions, sizes, etc. illustrated in the drawings in this specification are only used to match the contents disclosed in the specification so that people familiar with this technology can understand and read them, and are not used to limit the conditions for the implementation of the present application. Any structural modification, change in proportional relationship or adjustment of size, as long as the effect and purpose that can be produced by the present application are the same or similar, should still fall within the scope of the technical content disclosed in the present application. The specific features of the present application as disclosed herein, including, for example, specific dimensions, orientations, locations, and shapes will be determined in part by the particular intended application and use environment. Furthermore, in the embodiments described below, the same reference numerals may be used in common among different drawings to indicate the same parts or parts having the same functions, and their repeated descriptions may be omitted. In this specification, similar reference numerals and letters are used to denote similar items, and thus, once an item is defined in one figure, it need not be further discussed in subsequent figures.

It should be noted that, in this specification, relational terms such as first and second, etc. are merely used to distinguish one entity or operation from another entity or operation, and do not necessarily require or imply any actual relationship or order between these entities or operations. Furthermore, the terms "comprising", "including" or any other variations thereof are intended to cover non-exclusive inclusion, such that a process, method, article, or apparatus that includes a list of elements includes not only those elements but also other elements not expressly listed, or also includes elements inherent to such process, method, article, or apparatus. Without more constraints, an element defined by the phrase "comprising a..." does not exclude the existence of other identical elements in the process, method, article or apparatus comprising the element. The singular forms "one", "an" and "the" include plural objects, the term "or" is generally used in a sense including "and/or", the term "several" is generally used in a sense including "several", the term "at least two" is generally used in a sense including "two or more", and the term "multiple" is used in a sense including "two or more". In addition, the terms "first", "second" and "third" are used for descriptive purposes only and cannot be understood as indicating or suggesting relative importance or implicitly indicating the number of the indicated technical features.

In the description of the present application, it needs to be understood that the terms "center", "longitudinal", "lateral", "length", "width", "thickness", "up", "down", "front", "back", "left", "right", "vertical", "horizontal", "top", "bottom", "inside", "outside", "axial", "radial", "circumferential" and the like indicate orientations or positional relationships based on the orientations or positional relationships shown in the accompanying drawings, and are only for the convenience of describing the present application and simplifying the description, and do not indicate or imply that the referred device or element must have a specific orientation, be constructed and operated in a specific orientation, and therefore cannot be understood as limiting the present application.

In the description of the present application, unless otherwise clearly stipulated and limited, the terms "installed", "connected", "coupled" and "fixed" should be understood in a broad sense. For example, it can be a fixed connection, a detachable connection, or an integrated connection; it can be a mechanical connection or an electrical connection; it can be a direct connection or an indirect connection through an intermediate medium; it can be the internal connection of two elements or the interaction relationship between two elements. For those skilled in the art, the specific meanings of the above terms in the present application can be understood according to specific circumstances.

In the present application, unless otherwise clearly specified and limited, a first feature being "on" or "under" a second feature may include the first and second features being in direct contact, or the first and second features not being in direct contact but being in contact through another feature therebetween. Moreover, a first feature being "above", "on" and "over" a second feature may include the first feature being directly above and obliquely above the second feature, or simply means that the first feature is higher in horizontal height than the second feature. The first feature being "below", "beneath" and "below" the second feature may include the first feature being directly below and diagonally below the second feature, or simply means that the first feature is lower in horizontal height than the second feature.

The core idea of the present application is to provide a sensor applicator for percutaneous implantation of a sensor, and a medical device, so as to solve the problems of complex structure and high cost of the existing applicator. It should be noted that although this specification takes an example of the applicator being used to implant a sensor for detecting blood glucose concentration, as those skilled in the art can understand, the applicator can also be used to implant sensors for detecting parameters other than blood glucose concentration, and the present application is not limited to this. In addition, it should be noted that the distal end referred to in this specification refers to the end away from the skin of the target subject, and the proximal end refers to the end close to the skin of the target subject.

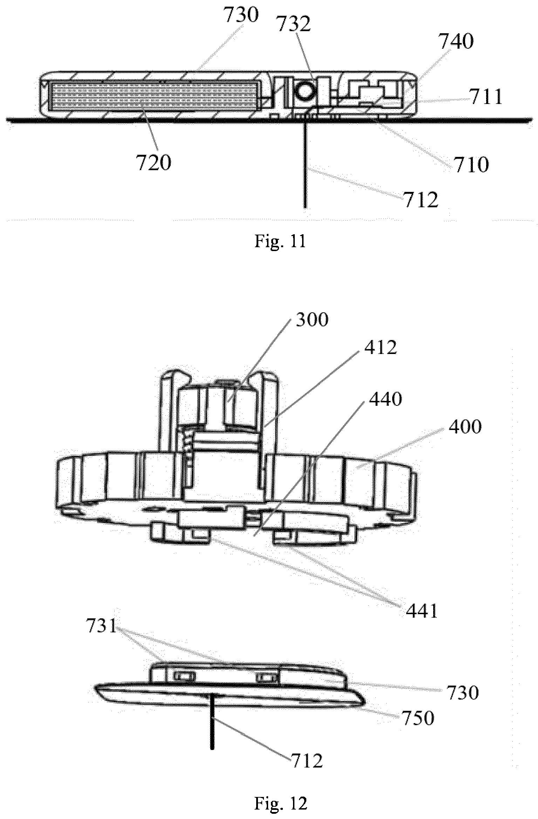

Please refer to Figs. 1 and 2, Fig. 1 is a schematic diagram showing an overall structure of a medical device according to an embodiment of the present application, and Fig. 2 is a cross-sectional view of the medical device before the pressing portion is triggered according to an embodiment of the present application. As shown in Figs. 1 and 2, the medical device includes an applicator and a sensor assembly 700. The sensor assembly 700 includes a sensor 710 (see Fig. 11) and a transmitter 720 (see Fig. 11), which are connected with each other. The applicator is configured to percutaneously implant the sensor 710 under the skin of the target subject, and the transmitter 720 is configured to receive a detection result from the sensor 710 and transmit the detection result to a terminal device (not shown in the figures). It should be noted that, as can be understood by those skilled in the art, the terminal device may be a mobile phone, a computer, a tablet computer or a receiver, and data transmission between the transmitter 720 and the terminal device may be achieved through existing communication modes, such as WiFi, Bluetooth, NFC or RFID.

Specifically, as shown in Fig. 2, the applicator includes a first housing 100 and a needle insertion assembly. The needle insertion assembly is arranged inside the first housing 100 and is configured to carry the sensor assembly 700. A plurality of pressing portions 200 are provided on the first housing 100. After the pressing portions 200 are triggered, the needle insertion assembly can move from a position away from the proximal end of the first housing 100 to a position close to the proximal end of the first housing 100, so that the sensor 710 can be percutaneously implanted under the skin of the target subject. Thus, by triggering the pressing portions 200, the needle insertion assembly can be moved from the distal end (i.e., a position away from the proximal end of the first housing 100) to a position close to the proximal end of the first housing 100, so that the sensor assembly 700 with the sensor 710 can be transported to the skin surface of the target subject, and the sensor 710 can be percutaneously implanted under the skin of the target subject to detect parameters such as the blood glucose concentration in the blood of the target subject.

Optionally, an anti-slip structure is provided on the outer wall of the first housing 100. The anti-slip structure includes one or more of ridges, bumps, recesses or textures arranged on the first housing. Therefore, as shown in Fig. 1, by providing a plurality of ridges 110 at the circumference of outer wall of the first housing 100, an anti-slip function is achieved. Thus, it is more convenient to use.

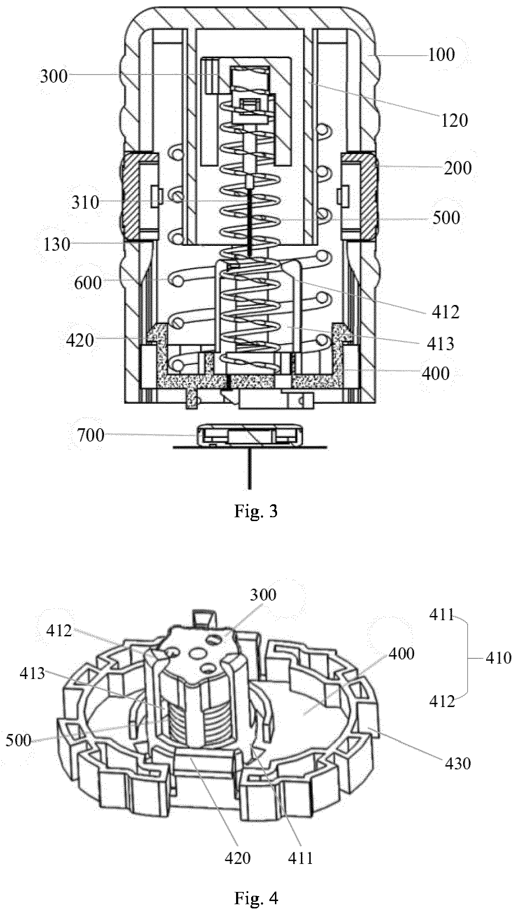

Please continue to refer to Figs. 2 and 3, Fig. 3 is a cross-sectional view of the applicator after the needle returning action is completed according to an embodiment of the present application. As shown in Figs. 2 and 3, the needle insertion assembly includes a needle holder 300 and a second housing 400. The needle holder 300 is configured to fix the implant needle 310. The distal end of the second housing 400 is provided with a first mounting seat 410 for releasably accommodating the needle holder 300. The proximal end of the second housing 400 is configured to be detachably connected to the sensor assembly 700. When the sensor 710 is percutaneously implanted under the skin of the target subject, the needle holder 300 can be released from the first mounting seat 410 and move toward the distal end of the first housing 100. The needle holder 300 is releasably accommodated in the first mounting seat 410. When the sensor 710 is percutaneously implanted under the skin of the target subject, the needle holder 300 can be released from the first mounting seat 410 and move toward the distal end of the first housing 100, so that the implant needle 310 together with the needle holder 300 can instantly rebound into the first housing 100, effectively avoiding the sharp implant needle 310 from accidentally injuring the operator or other personnel. In addition, the sensor assembly 700 is detachably connected to the second housing 400. Therefore, it is easier to remove the applicator after the needle returning action is completed, and the sensor assembly 700 can retain on the skin of the target subject to achieve continuous monitoring of parameters such as blood glucose concentration. In summary, the applicator according to the present application is not only simple in structure and low in cost, but also easier to operate.

Further, as shown in Figs. 2 and 3, a first elastic member 500 is provided in the first mounting seat 410 along the axial direction thereof. The distal end of the first elastic member 500 is connected to the needle holder 300, and the proximal end of the first elastic member 500 is connected to the proximal end of the first mounting seat 410. Before the needle insertion assembly is moved to a position close to the proximal end of the first housing 100, the first elastic member 500 is in a compressed state so that the needle holder 300 can be accommodated in the first mounting seat 410. After the needle insertion assembly is moved to a position close to the proximal end of the first housing 100, under the action of the first elastic member 500, the needle holder 300 can be released from the first mounting seat 410 and move toward the distal end of the first housing 100. Therefore, after the needle insertion assembly is moved to a position close to the proximal end of the first housing 100 (i.e., after the sensor 710 is implanted under the skin of the target subject), the needle holder 300 can be driven to be released from the first mounting seat 410 and moved to a position close to the distal end of the first housing 100 under the resilience force of the first elastic member 500, thereby effectively preventing the sharp implant needle 310 from accidentally injuring the operator or other personnel during and after the applicator is removed, thereby improving the safety of the medical device during use according to the present application.

As shown in Figs. 2 and 3, the first elastic member 500 is optionally a spring, and the first elastic member 500 is arranged around the implant needle 310. Before the needle insertion assembly is moved to a position close to the proximal end of the first housing 100, the proximal end of the implant needle 310 protrudes out of the first elastic member 500. Therefore, by using a spring as the first elastic member 500, the overall structure of the applicator according to the present application can be further simplified, and the cost can be effectively reduced. It should be noted that, as those skilled in the art can understand, the first elastic member 500 may also be implemented as other elastic elements than a spring, and the present application is not limited to this.

Furthermore, please refer to Figs. 2 to 4, Fig. 4 is a schematic diagram showing a structure of a needle insertion assembly before returning of the needle according to an embodiment of the present application. As shown in Figs. 2 to 4, the first mounting seat 410 includes a hollow base 411 and a plurality of (for example, three, but not limited to three) elastic claws 412 extending along the axial direction of the base 411 toward the distal end of the first housing 100. A first accommodation space 413 configured to accommodate the needle holder 300 and the first elastic member 500 can be formed between the base 411 and the elastic claws 412. Before the needle insertion assembly is moved to a position close to the proximal end of the first housing 100, the distal end of each elastic claw 412 can abut against the distal end of the needle holder 300 to accommodate the needle holder 300 in the first accommodation space 413. After the needle insertion assembly is moved to a position close to the proximal end of the first housing 100, under the action of the first elastic member 500, the distal ends of each elastic claws 412 can be separated from the needle holder 300, so that the needle holder 300 can be released from the first mounting seat 410 and move toward the distal end of the first housing 100. It should be noted that, as can be understood by those skilled in the art, in some other embodiments, the first mounting seat 410 may not include the base 411 but only include a plurality of elastic claws 412. In this case, the first accommodation space 413 is surrounded by the plurality of elastic claws 412.

Specifically, the resilience force of the first elastic member 500 is greater than the deformation force of the plurality of elastic claws 412. Therefore, before the needle insertion assembly is moved to a position close to the proximal end of the first housing 100 (i.e., before the sensor 710 is implanted under the skin of the target subject), under the action of an external force, the downward pressure of the elastic claws 412 on the needle holder 300 can reach a balance with the resilience force of the first elastic member 500, so that the needle holder 300 can be firmly fixed in the first mounting seat 410; after the needle insertion assembly is moved to a position close to the proximal end of the first housing 100 (i.e., after the sensor 710 is implanted under the skin of the target subject), under the action of the resilience force of the first elastic member 500 due to the disappearance of the external force, the needle holder 300 can be freed from the restraint of the elastic claws 412, and can then be released from the first mounting seat 410 and move toward the distal end of the first housing 100. It should be noted that, although this specification uses the example of the first mounting seat 410 including three elastic claws 412 for explanation, as those skilled in the art can understand, in some other embodiments, the first mounting seat 410 may also include two, four or more elastic claws 412, or even elastic arms that can restore deformation to the four sides. The elastic claws can be specifically configured based on actual conditions, and the present application is not limited to this.

Optionally, as shown in Figs. 2 and 3, a hollow clasping member 120 is arranged in the first housing 100 along the axial direction thereof. Before the needle insertion assembly is moved to a position close to the proximal end of the first housing 100, the clasping member 120 can be sleeved over the elastic claws 412 to clasp the elastic claws 412. After the needle insertion assembly is moved to a position close to the proximal end of the first housing 100, the clasping member 120 can be separated from the elastic claws 412 so that the distal ends of the elastic claws 412 can be separated from the needle holder 300, and then the first elastic member 500 instantly causes the needle holder 300 to automatically move toward the distal end of the first housing 100. Before the needle insertion assembly is moved to a position close to the proximal end of the first housing 100 (i.e., before the sensor 710 is implanted under the skin of the target subject), the clasping member 120 can be sleeved over the elastic claws 412, so that the elastic claws 412 can be tightly restrained to prevent it from being deformed. Therefore, the distal ends of the elastic claws 412 can abut against the needle holder 300 to accommodate the needle holder 300 in the first mounting seat 410. After the needle insertion assembly is moved to a position close to the proximal end of the first housing 100 (i.e., before the sensor 710 is implanted under the skin of the target subject), the distal ends of the elastic claws 412 are moved to be separated from the clasping member 120, and the elastic claws 412 are no longer restrained by the clasping member 120. In this case, the resilience force of the first elastic member 500 is greater than the deformation force of the plurality of elastic claws 412, under the action of the resilience force of the first elastic member 500, the elastic claws 412 can bend or eject outward. Therefore, the needle holder 300 can get rid of the restraint of the elastic claws 412 and then is released.

Please continue to refer to Figs. 2 and 3. As shown in Figs 2 and 3, a second elastic member 600 is arranged in the first housing 100 along the axial direction thereof. The distal end of the second elastic member 600 is connected to the distal end of the first housing 100, and the proximal end of the second elastic member 600 is connected to the second housing 400. Before the pressing portion 200 is triggered, the second elastic member 600 is in a compressed state. After the pressing portion 200 is triggered, under the action of the second elastic member 600, the second housing 400 can move toward the position where the proximal end of the first housing 100 is. Since the second elastic member 600 is in a compressed state before the pressing portion 200 is triggered, after the pressing portion 200 is triggered, the second housing 400 can be moved more smoothly toward the proximal end of the first housing 100 under the action of the resilience force of the second elastic member 600, which enables the applicator according to the present application to smoothly and accurately implant the sensor 710 under the skin of the target subject, thereby improving the stability of the medical device during use according to the present application.

Optionally, as shown in Figs. 2 and 3, the second elastic member 600 is a spring, and the second elastic member 600 is arranged to surround the clasping member 120 and the first mounting seat 410. Therefore, by using a spring as the second elastic member 600, the overall structure of the applicator according to the present application can be further simplified, and the cost can be effectively reduced. In addition, since the second elastic member 600 is arranged to surround the clasping member 120 and the first mounting seat 410, not only can the internal space of the applicator be fully utilized and the overall structure of the applicator according to the present application be further simplified, but also the flexibility of the applicator during use can be further improved, which is conducive to more quickly implanting the sensor 710 under the skin of the target subject. It should be noted that, as those skilled in the art can understand, the second elastic member 600 may also be other elastic members than a spring, and the present application is not limited to this.

Furthermore, as shown in Figs. 2 to 4, a plurality of abutment members 420 are provided at the distal end of the second housing 400, and an abutment platform 130 matched with the abutment members 420 is provided on the inner wall of the first housing 100, and the abutment platform 130 is close to the position where the pressing portion 200 is. Before the pressing portion 200 is triggered, the abutment members 420 abut on the abutment member 130, and after the pressing portion 200 is triggered, the abutment members 420 can be detached from the abutment platform 130. Since the abutment platform 130 is close to the position where the pressing portion 200 is, when the pressing portion 200 is pressed inward toward the interior of the first housing 100 and triggered, the inwardly moving pressing portion 200 can push the abutment member 420 to disengage from the abutment platform 130, thereby allowing the second housing 400 to move relative to the first housing 100. Therefore, the needle insertion assembly can move from a position away from the proximal end of the first housing 100 to a position close to the proximal end of the first housing 100. As a result, the sensor 710 can be implanted under the skin of the target subject.

Optionally, as shown in Figs. 2 to 4, a pair of pressing portions 200 is disposed opposite to each other on the first housing 100, and a pair of abutment members 420 is disposed on the second housing 400. The abutment members 420 are elastic members, and the abutment members 420 is disposed in such a way that each of them corresponds with one of the pressing portions 200. Therefore, by providing two pairs of pressing portions 200 and abutment members 420 that are disposed in such a way that each of the abutment members 420 corresponds with one of the pressing portions 200, it can be ensured that the needle insertion assembly can be firmly fixed in the first housing 100 before the pressing portion 200 is triggered. It should be noted that, although this specification uses the example that the first housing 100 includes two pressing portions 200 and the second housing 400 is provided with two abutment members 420 for explanation, as those skilled in the art can understand, in some other embodiments, the first housing 100 may be provided with one pressing portion 200, three pressing portions 200 or more pressing portions 200, and the second housing 400 may be correspondingly provided with one abutment member 420, three abutment members 420 or more abutment members 420, and the present application is not limited to this.

Furthermore, as shown in Figs. 2 to 4, an inclined surface 421 is provided on a side of the abutment member 420 close to the pressing portion 200. Therefore, this arrangement can further facilitate the installation of the second housing 400 from the proximal end of the first housing 100, making it easier to assemble.

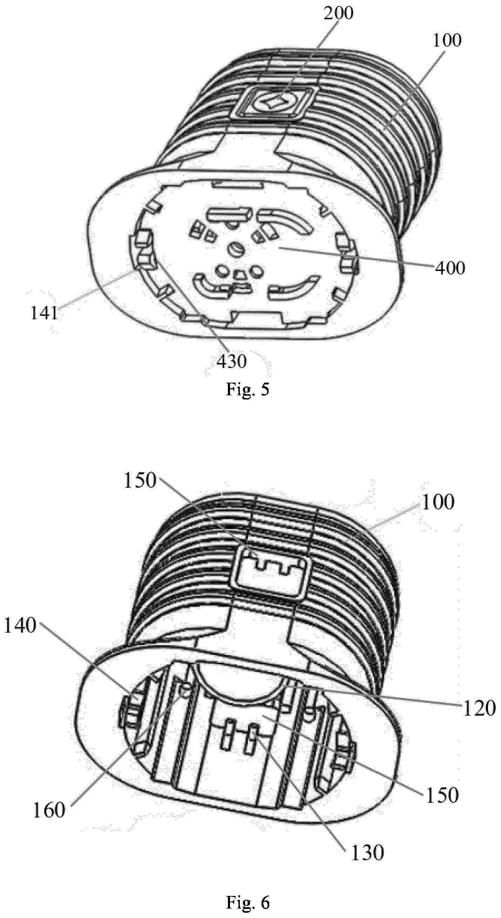

Optionally, please refer to Figs. 4 to 6, Fig. 5 is a schematic diagram showing a three-dimensional view of the structure of the applicator after the needle returning action is completed according to an embodiment of the present application, and Fig. 6 is a schematic diagram of a first housing 100 according to an embodiment of the present application. As shown in Figs. 4 to 6, sliding slots 430 extending along the axial direction of the second housing 400 are respectively provided on both sides of the second housing 400, and the sliding slots 430 extend through the proximal end and the distal end of the second housing 400. Slide rails 140 matched with the sliding slots 430 are provided on the inner wall of the first housing 100. Therefore, by providing sliding slots 430 on both sides of the second housing 400 and providing slide rails 140 on the inner wall of the first housing 100, it can be ensured that after the pressing portion 200 is triggered, the sliding slots 430 can slide along the slide rails 140, thereby ensuring that the needle insertion assembly can smoothly move from a position away from the proximal end of the first housing 100 to a position close to the proximal end of the first housing 100. Therefore, the applicator according to the present application can smoothly and accurately implant the sensor 710 under the skin of the target subject. As a result, the stability of the medical device during use is improved according to the present application.

Furthermore, as shown in Fig. 5, a stopper 141 is provided at the proximal end of each slide rail 140. Therefore, when the needle insertion assembly is moved to a position close to the proximal end of the first housing 100, the stopper 141 can prevent the needle insertion assembly from moving further downward and disengaging from the first housing 100, thereby ensuring that the applicator can be more easily removed from the skin of the target subject after the needle returning action is completed. In addition, since the stopper 141 can prevent the needle insertion assembly from detaching from the first housing 100, it can prevent the implant needle 310 from being exposed due to the detachment of the second housing 400 from the first housing 100, further reducing the risk of accidental injury by the implant needle 310.

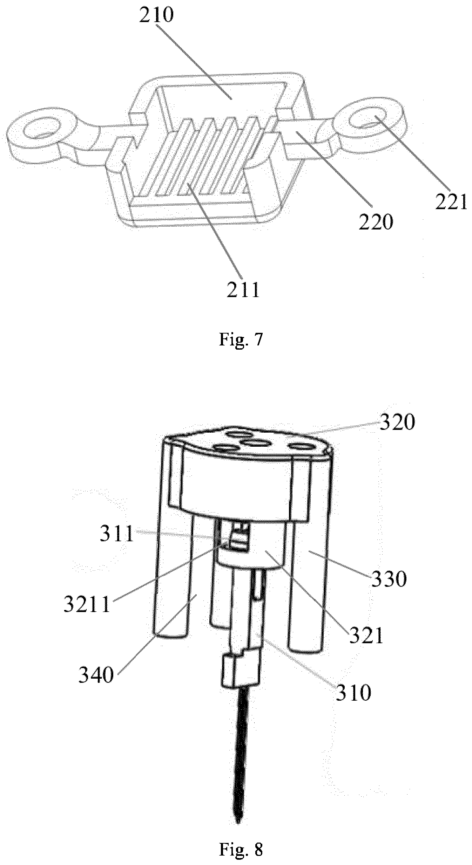

Please continue to refer to Figs. 6 and 7, Fig. 7 is a schematic diagram showing the structure of a pressing portion 200 according to an embodiment of the present application. As shown in Figs. 6 and 7, the pressing portion 200 includes a main body 210 and first cantilevers 220 arranged on both sides of the main body 210. The first cantilevers 220 extend outward along the length direction of the main body 210. The first cantilevers 220 are connected to the inner wall of the first housing 100. A first mounting hole 150 corresponding to the main body 210 is defined on the first housing 100. The main body 210 is installed in the first mounting hole 150 so that the main body 210 can move back and forth in the first mounting hole 150. Therefore, the pressing portion 200 can be more conveniently fixed to the first housing 100 by welding or other connection methods through the first cantilevers 220, thereby effectively preventing the pressing portion 200 from being separated from the first housing 100 when the pressing portion 200 is pressed. It should be noted that, as those skilled in the art can understand, the first cantilevers 220 each has a certain elasticity, so that when the operator's hands leave the pressing portion 200, the pressing portion 200 can smoothly return to its initial shape.

Optionally, as shown in Fig. 7, each first cantilever 220 is arranged in an arc shape, a second mounting hole 221 is defined on a side of the first cantilever 220 away from the main body 210, and a mounting pillar 160 matching with the second mounting hole 221 is provided on the inner wall of the first housing 100. Since the first cantilever 220 is arranged in an arc shape, it is more convenient for the pressing portion 200 to smoothly return to the initial shape when the external force is removed. In addition, since a second mounting hole 221 is defined on a side of the first cantilever 220 and a mounting pillar 160 is provided on the inner wall of the first housing 100, the second mounting hole 221 can be firmly mounted on the mounting pillar 160 by welding or other connection methods so that the pressing portion 200 is fixed to the first housing 100.

Optionally, as shown in Fig. 7, the main body 210 has a hollow structure. An opening is defined on a side of the main body 210 close to the inner wall of the first housing 100, and a plurality of ribs 211 are provided on the inner wall of the main body 210 along the axial direction thereof. Therefore, by configuring the main body 210 to have a hollow structure, the weight of the pressing portion 200 can be reduced, further reducing the cost. Since an opening is defined on a side of the main body 210 close to the inner wall of the first housing 100, not only can the cost be further reduced, but also the abutment platform 130 can be arranged more conveniently. In addition, since a plurality of ribs 211 are provided on the inner wall of the main body 210, the ribs 211 can strengthen the main body 210, thereby improving the stability of the overall structure of the pressing portion 200.

Please continue to refer to Fig. 8, which is a schematic diagram showing the structure of a needle holder 300 according to an embodiment of the present application. As shown in Fig. 8, the needle holder 300 includes a fixing seat 320 and a plurality of guide pillars 330 extending along the axial direction of the fixing seat 320 toward the proximal end of the first housing 100. An opening is defined at the proximal end of the fixing seat 320, and a connecting portion 321 is arranged in the fixing seat 320 along the axial direction thereof. The distal end of the implant needle 310 is provided with a plurality of (for example, two, but not limited to two) first clamping portions 311, and the connecting portion 321 is provided with first clamping slots 3211 that match with the first clamping portions 311. Therefore, the implant needle 310 can be more easily fixed in the needle holder 300 by inserting the first clamping portions 311 into the first clamping slots 3211. In addition, as shown in Figs. 2 and 8, a second accommodation space 340 for accommodating the first elastic member 500 can be formed between the fixing seat 320 and the guide pillars 330. As a result, not only can the internal volume of the applicator be further fully utilized and the overall structure of the applicator according to the present application be further simplified, but it can also make it easier to install the first elastic member 500. It should be noted that, although this specification uses the example that the implant needle 310 includes two first clamping portions 311 and the connecting portion 321 includes two first clamping slots 3211 for explanation, as those skilled in the art can understand, in some other embodiments, the implant needle 310 may include one first clamping portion 311, three first clamping portions 311 or more first clamping portions 311, and the connecting portion 321 may be provided with one first clamping slot 3211, three first clamping slots 3211 or more first clamping slots 3211, and the present application is not limited to this.

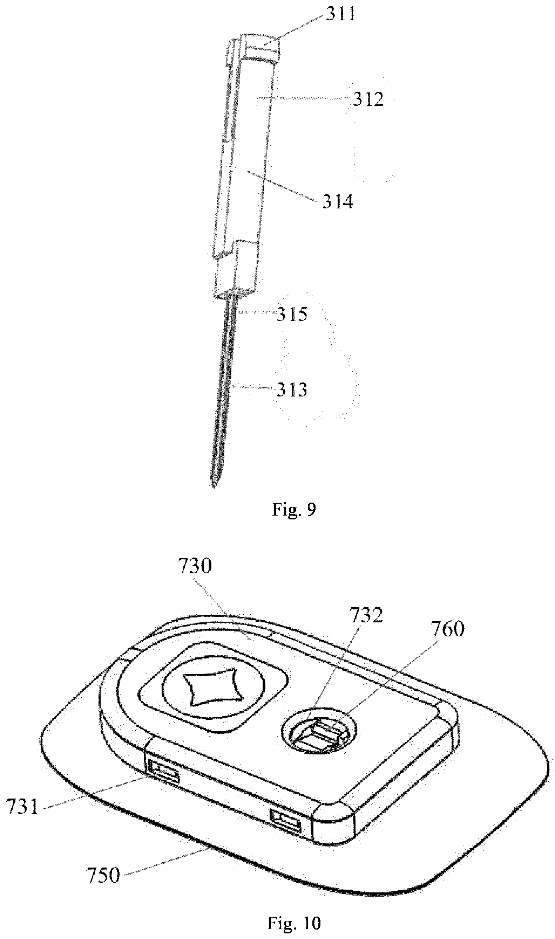

Further, please refer to Fig. 9, which is a schematic diagram showing the structure of an implant needle 310 according to an embodiment of the present application. As shown in Fig. 9, a plurality of (for example, two) second cantilevers 312 are provided at the distal end of the implant needle 310. The second cantilevers 312 extend outwardly along the axial direction of the implant needle 310, and the first clamping portions 311 are provided at the distal ends of the second cantilevers 312. Since the second cantilevers 312 each has a certain elasticity, by providing the second cantilevers 312 on which the first clamping portions 311 are arranged, it is easier to clamp the first clamping portions 311 into the first clamping slots 3211, thereby facilitating the assembly of the applicator.

Please continue to refer to Figs. 2, 10 and 11, Fig. 10 is a schematic diagram showing the structure of a sensor assembly 700 according to an embodiment of the present application, and Fig. 11 is a cross-sectional view of the sensor assembly 700 according to an embodiment of the present application. As shown in Figs. 2, 10 and 11, the sensor assembly 700 also includes a foundation 730, a sensor 710 and a transmitter 720. The sensor 710 and the transmitter 720 are installed on the foundation 730. The proximal end of the second housing 400 is provided with a second mounting seat 440 configured to releasably accommodate the foundation 730. The foundation 730 is detachably connected to the second mounting seat 440. Therefore, by integrating the sensor 710 and the transmitter 720 on the same foundation 730, the structure of the sensor assembly 700 can be simplified, thereby further simplifying the overall structure of the medical device according to the present application. In addition, since the second housing 400 is provided with a second mounting seat 440, the sensor assembly 700 can be installed in the applicator by installing the foundation 730 in the second mounting seat 440. In addition, since the foundation 730 and the second mounting seat 440 are detachably connected, it is easier to separate the applicator from the sensor assembly 700 after the sensor 710 is implanted under the skin of the target subject, so as to remove the applicator and leave the sensor assembly 700 on the skin of the target subject, thereby achieving continuous monitoring of parameters such as blood glucose concentration.

Specifically, as shown in Fig. 11, a circuit board 740 is provided on the sensor assembly 700, and both the sensor 710 and the transmitter 720 are electrically connected to the circuit board 740. Thus, the data detected by the sensor 710 can be transmitted to the transmitter 720 through the circuit board 740, so that the detected data can be transmitted to an external terminal device from the transmitter 720. More specifically, a conductive element 711 is disposed on the sensor 710. Thus, the sensor 710 can be electrically connected to the circuit board 740 by the conductive element 711. It should be noted that, as those skilled in the art can understand, the foundation 730 is also provided with a battery, so that the sensor 710 and the transmitter 720 can be powered by the battery.

Further, please refer to Fig. 12, which is a schematic diagram showing a structure where the second housing 400 and the sensor assembly 700 are separated from each other according to an embodiment of the present application (in which the implant needle is not shown). As shown in Fig. 12, a plurality of second clamping slots 731 are formed on the outer periphery of the foundation 730, and second clamping portions 441 matched with the second clamping slots 731 are formed on the inner wall of the second mounting seat 440. Therefore, when the second clamping portions 441 are inserted into the second clamping slots 731, the sensor assembly 700 can be firmly fixed on the second housing 400, so that the applicator can smoothly transport the sensor assembly 700 to the skin of the target subject during the movement of the needle insertion assembly toward the proximal end of the first housing 100. As a result, the sensor 710 can be implanted under the skin of the target subject. When the second clamping portions 441 are disengaged from the second clamping slots 731 due to external force, the sensor assembly 700 can be smoothly separated from the second housing 400. As a result, the applicator can be smoothly removed, and the sensor assembly 700 can be retained on the skin of the target subject. It can be seen that by using the second clamping slot 731 and the second clamping portion 441 that are matched with each other, the detachable connection between the applicator and the sensor assembly 700 can be established smoothly.

Optionally, as shown in Figs. 10 and 12, the proximal end of the foundation 730 is provided with a contact surface 750 extending outwardly along the circumferential direction of the foundation 730 for contacting the skin of the target subject, and an adhesive is provided on the contact surface 750. Thus, by providing an adhesive on the contact surface 750, the sensor assembly 700 can be firmly adhered to the skin of the target subject after the sensor assembly 700 is transported to a position close to the skin of the target subject. When the applicator is removed, the adhesive force of the adhesive on the skin is greater than the holding force of the second clamping portions 441 on the sensor assembly 700, so that the sensor assembly 700 can be retained on the skin of the target subject after the applicator is removed. In addition, since the contact surface 750 extends outward relative to the foundation 730, the area of the contact surface 750 can be effectively increased, and the adhesion between the adhesive and the skin of the target subject can be further improved. It should be noted that, as can be understood by those skilled in the art, in some other embodiments, the sensor assembly 700 may be attached to the skin of the target subject in any other common manner.

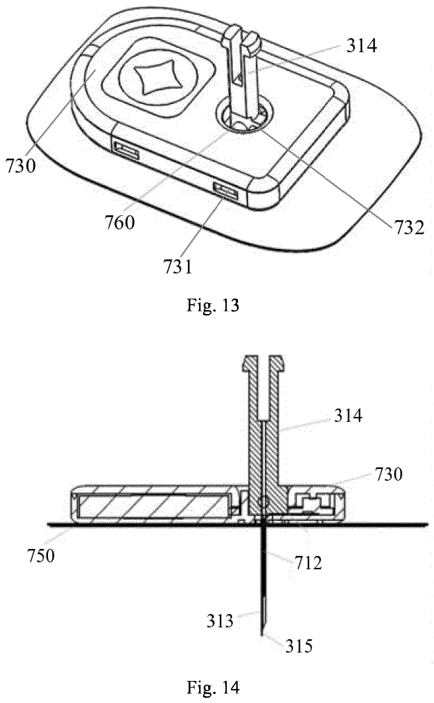

Further, please refer to Figs. 9, 10, 13 and 14, Fig. 13 is a schematic diagram showing the connection between the sensor assembly 700 and the implant needle 310 according to an embodiment of the present application, and Fig. 14 is a cross-sectional view of Fig. 13. As shown in Figs. 9, 10, 13 and 14, a through hole 732 allowing the implant needle 310 to pass therethrough is defined on the foundation 730. The proximal end of the sensor 710 is provided with a pin 712 protruding out of the through hole 732. A groove 313 configured to wrap the pin 712 is defined on the implant needle 310 along the axial direction thereof. Therefore, when the needle insertion assembly is moved to a position close to the proximal end of the first housing 100, under the action of the implant needle 310, the proximal end of the pin 712 can be smoothly implanted under the skin of the target subject to collect parameters such as blood glucose concentration.

Optionally, as shown in Figs. 10 and 13, an elastic contact switch 760 is provided in the through hole 732. When the needle holder 300 is fixed in the first mounting seat 410, the elastic contact switch 760 is in a non-conducting state. When the needle holder 300 is released from the first mounting seat 410 and moves to a position close to the distal end of the first housing 100, the elastic contact switch 760 is in a conducting state. Therefore, by providing the elastic contact switch 760, there is no need to provide an additional switch on the sensor assembly 700, and there is no need to perform software operation control. After the needle returning action is completed, the sensor assembly 700 can automatically activate the signal transmission function, thereby avoiding power consumption during storage after leaving factory. This can not only further simplify the structure of the sensor assembly 700, enabling the sensor assembly 700 to be smaller in size and more convenient to wear, but also can further reduce the operating steps, making it easier to operate and ensuring the service life of the sensor assembly 700.

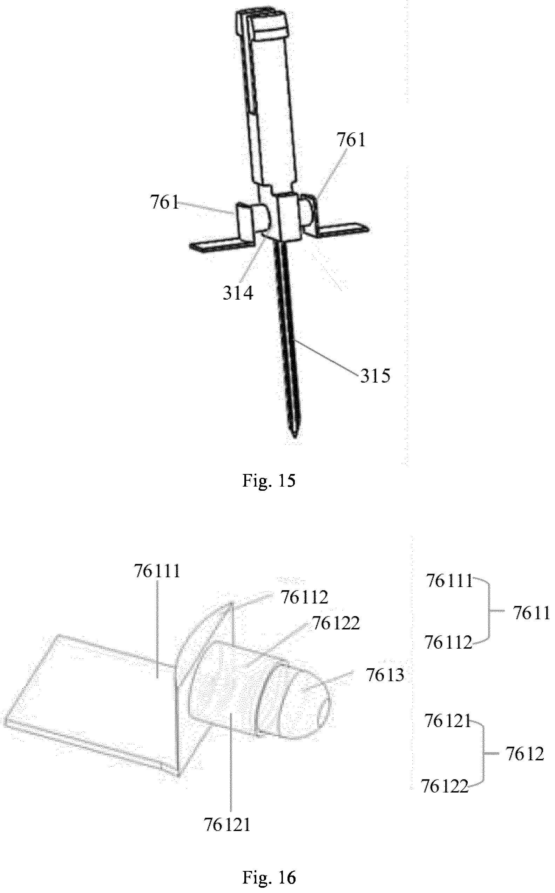

Specifically, please refer to Fig. 15, which is a schematic diagram of an elastic contact switch in its non-conducting state according to an embodiment of the present application. As shown in Fig. 15, the elastic contact switch 760 includes a pair of conductive members 761 arranged opposite to each other. At least one of the two conductive members 761 is elastic. The implant needle 310 includes a needle handle 314 and a needle body 315 connected to each other. The needle handle 314 is made of insulating material. When the needle holder 300 is accommodated in the first mounting seat 410, one of the conductive members 761 abuts against one side of the needle handle 314, and the other one of the conductive members 761 abuts against the other side of the needle handle 314, so that the elastic contact switch 760 is in a non-conducting state. When the needle holder 300 is released from the first mounting seat 410 and moves to a position close to the distal end of the first housing 100, the two conductive members 761 can contact each other, so that the elastic contact switch 760 is in a conducting state. Thus, when the needle holder 300 is accommodated in the first mounting seat 410, the elastic contact switch 760 can be in a non-conducting state because the two conductive members 761 are separated by the insulating needle handle 314 (i.e., the elastic conductive member 761 is in a compressed state). At this time, the circuit in the sensor assembly 700 is not closed, and the transmitter 720 and the sensor 710 cannot work. When the needle holder 300 is released from the first mounting seat 410 and moves to a position close to the distal end of the first housing 100 (i.e., after the needle returning action is completed), the implant needle 310 withdraws from the sensor assembly 700, so that the two conductive members 761 can contact each other under the action of the elastic force, and the elastic contact switch 760 is in a conducting state. At this time, the proximal end of the pin 712 is smoothly implanted under the skin of the target subject, the circuit in the sensor assembly 700 is closed, and the transmitter 720 and the sensor 710 start to work.

Please continue to refer to Fig. 16, which is a schematic diagram showing the structure of a conductive member 761 which is elastic according to an embodiment of the present application. As shown in Fig. 16, in this embodiment, the elastic conductive member 761 includes a connecting plate 7611, a telescopic member 7612 and a contact terminal 7613. The telescopic member 7612 is fixed on the connecting plate 7611, and the contact terminal 7613 is connected to the end of the telescopic member 7612 away from the connecting plate 7611. Under the action of the telescopic member 7612, the contact terminal 7613 can move in a direction away from the connecting plate 7611. Specifically, when the implant needle 310 is withdrawn from the sensor assembly 700, under the action of the telescopic member 7612, the contact terminal 7613 of the conductive member 761 can move away from the connecting plate 7611 until the conductive member 761 contacts another conductive member 761, so that the elastic contact switch 760 is conducting.

Further, as shown in Fig. 16, the telescopic member 7612 includes a hollow sleeve 76121 and a third elastic member 76122 arranged in the sleeve 76121, and the sleeve 76121 is fixed on the connecting plate 7611. One end of the third elastic member 76122 is connected to the sleeve 76121, and the other end of the third elastic member 76122 is connected to the contact terminal 7613. When the needle holder 300 is fixed in the first mounting seat 410, the third elastic member 76122 is in a compressed state. When the implant needle 310 is withdrawn from the sensor assembly 700, under the action of the resilience force of the third elastic member 76122, the contact terminal 7613 can move toward a position away from the connecting plate 7611. Optionally, the third elastic member 76122 is a spring. Thus, by using a spring as the third elastic member 76122, the structure of the sensor assembly 700 can be further simplified, thereby effectively reducing the cost. It should be noted that, as can be understood by those skilled in the art, in some other embodiments, the telescopic member 7612 may not include the sleeve 76121 but only include a third elastic member 76122. In this case, one end of the third elastic member 76122 is connected to the connecting plate 7611, and the other end is connected to the contact terminal 7613. By selecting a third elastic member 76122 with conductive properties, electrical conduction between the contact terminal 7613 and the connecting plate 7611 can be achieved.

Furthermore, as shown in Fig. 16, the connecting plate 7611 includes a first plate body 76111 and a second plate body 76112 connected to each other. The first plate body 76111 is arranged along the radial direction of the through hole 732, the second plate body 76112 is arranged along the axial direction of the through hole 732, and the telescopic member 7612 is installed on a side of the second plate body 76112 away from the first plate body 76111. Therefore, this arrangement facilitates the installation of the conductive member 761.

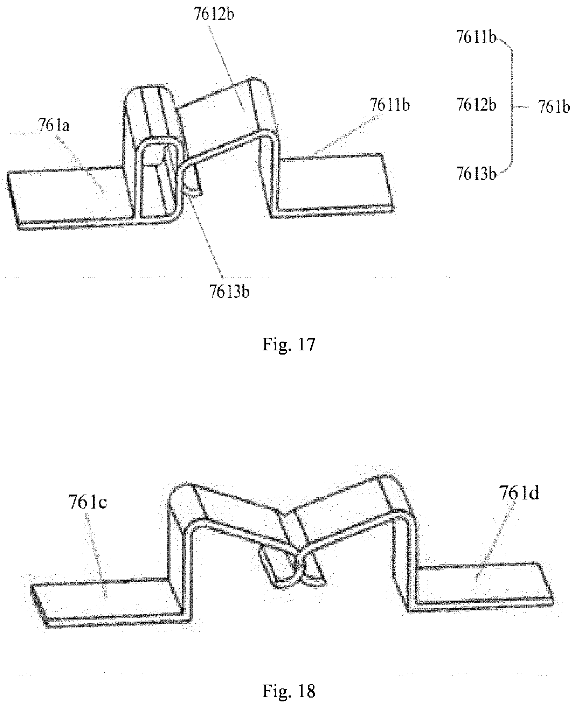

Please continue to refer to Fig. 17, which is a schematic diagram of an elastic contact switch 760 in its conducting state according to an embodiment of the present application. As shown in Fig. 17, in this embodiment, the elastic contact switch 760 includes a conductive member 761a and a conductive member 761b. The conductive member 761a is not elastic. Therefore, after the needle returning action is completed, the conductive member 761a remains stationary. The conductive member 761b is elastic. Therefore, after the needle returning action is completed, the conductive member 761b can move close to the conductive member 761a and contact the conductive member 761a, so that the elastic contact switch 760 is conducting. Specifically, as shown in Fig. 17, in this embodiment, the conductive member 761b includes a connecting plate 7611b, a telescopic member 7612b and a contact terminal 7613b. The elastic conductive member 761b provided in this embodiment is different from the elastic conductive member 761 provided in the previous embodiment. In this embodiment, the telescopic member 7612b and the contact terminal 7613b are structured integrally, the telescopic member 7612b is an inclined spring piece, and the contact terminal 7613b is formed by bending one end of the telescopic member 7612b that is away from the connecting plate 7611b. Since the telescopic member 7612b and the contact terminal 7613b are structured integrally, the structure of the sensor assembly 700 can be further simplified, thereby effectively reducing the cost.

Please continue to refer to Fig. 18, which is a schematic diagram of an elastic contact switch 760 in its conducting state according to another embodiment of the present application. As shown in Fig. 18, in this embodiment, the elastic contact switch 760 includes a conductive member 761c and a conductive member 761d. The elastic contact switch 760 provided in this embodiment is different from the elastic contact switch 760 provided in the previous embodiment. In this embodiment, the conductive member 761c and the conductive member 761d are both elastic, and the structures of the conductive member 761c and the conductive member 761d are similar to the structure of the conductive member 761b in the previous embodiment, so they will not be described again here.

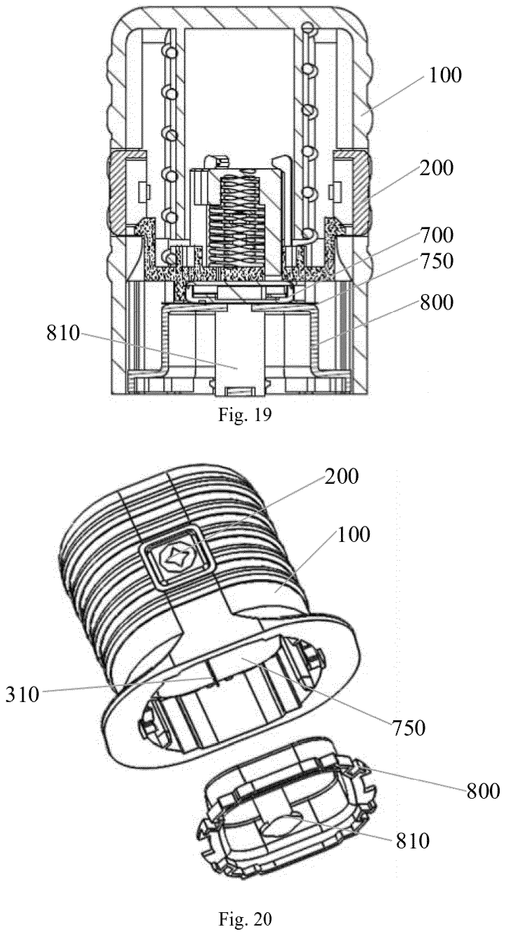

Please continue to refer to Figs. 19 and 20, Fig. 19 is a schematic diagram showing an overall structure of a medical device according to another embodiment of the present application, and Fig. 20 is a schematic diagram showing an exploded view of the structure of the medical device shown in Fig. 19. As shown in Figs. 19 and 20, the medical device also includes a protective cover 800. The sensor assembly 700 and the needle insertion assembly are enclosed in a space enclosed by the first housing 100 and the protective cover 800. The protective cover 800 is detachably installed at the proximal end of the first housing 100. Therefore, by providing the protective cover 800, the sensor assembly 700 and the needle insertion assembly can be sealed inside the first housing 100, which can not only effectively prevent the needle insertion assembly and the sensor assembly 700 from being contaminated before using the medical device, but also effectively prevent the sharp implant needle 310 from accidentally injuring the operator or other personnel due to the accidental triggering of the pressing portion 200 before removing the protective cover 800. It should be noted that, as can be understood by those skilled in the art, before removing the protective cover 800, under the action of the protective cover 800, pressing the pressing portion 200 will not cause the needle insertion assembly to move from a position away from the proximal end of the first housing to a position close to the proximal end of the first housing; only when the protective cover 800 is removed, can the pressing portion 200 be pressed to move the needle insertion assembly from a position away from the proximal end of the first housing 100 to a position close to the proximal end of the first housing 100, so as to percutaneously implant the sensor 710 under the skin of the target subject. In addition, it should be noted that, as those skilled in the art can understand, a snap-on connection or any other detachable connection commonly used in the prior art can be used as the detachable connection between the protective cover 800 and the first housing 100, and the present application is not limited to this.

Further, as shown in Figs. 19 and 20, when an adhesive is provided on the contact surface 750 of the sensor assembly 700, the release liner of the tape cooperates with the distal end of the protective cover 800, and the cooperation can be achieved through double-sided tape or through mechanical connection. Therefore, the release liner can protect the adhesive and ensure the effectiveness of the adhesive. When the protective cover 800 is pulled outward, the release liner can move along with the protective cover 800, so that the release liner can be separated from the adhesive.

Furthermore, as shown in Figs. 19 and 20, the protective cover 800 is provided with a protective seat 810 corresponding to the implant needle 310. Therefore, when the protective cover 800 is installed inside the first housing 100, the implant needle 310 wraps the pin 712 can be inserted into the protective seat 810, thereby sealing the implant needle 310 and the pin 712, effectively preventing the sharp implant needle 310 from accidentally injuring the operator or other personnel.

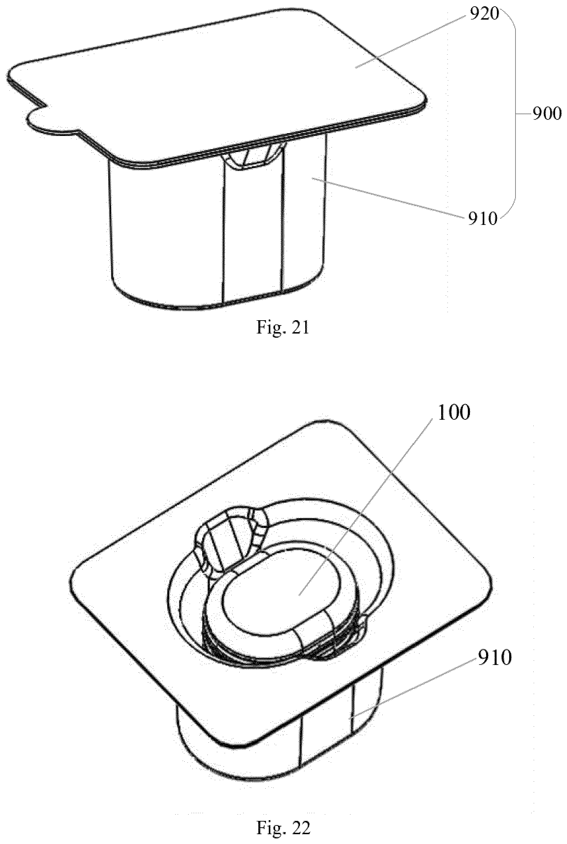

Please continue to refer to Figs. 21 and 22, Fig. 21 is a schematic diagram showing an overall structure of a medical device according to still another embodiment of the present application, and Fig. 22 is a schematic diagram showing a partial structure of the medical device shown in Fig. 21 (after the box cover is removed). As shown in Figs. 21 and 22, the medical device further includes a sterilization box 900, and the sensor assembly 700 and the applicator are sterilized as a whole and installed in the sterilization box. Therefore, by covering the first housing 100 with the sterilization box 900, the needle insertion assembly, the sensor assembly 700 and other components of the medical device can be placed in a sterile environment before use, further preventing the needle insertion assembly, the sensor assembly 700 and other components from being contaminated. Specifically, as shown in Figs. 21 and 22, the sterilization box 900 includes a box body 910 and a box cover 920 covered on the box body 910. When the box cover 920 is opened (i.e., the box cover 920 is removed), the applicator and sensor assembly 700 located in the box body 910 can be taken out without further assembly. The corresponding operation can be performed to percutaneously implant the sensor 710 under the skin of the target subject, which is convenient and quick, and greatly avoids the contamination problem during the percutaneous implantation process. In addition, it should be noted that, as can be understood by those skilled in the art, only a portion of the sensor assembly 700 (including at least the sensor 710) can be assembled with the applicator and installed in the sterilization box 900. After the percutaneous implantation is completed, the sensor 710 can be assembled with other parts of the sensor assembly 700 (such as the transmitter 720) to detect parameters such as the blood glucose concentration in the blood of the target subject. The present application is not limited to this.

In summary, compared with the prior art, the sensor applicator for percutaneous implantation of a sensor, and a medical device according to the present application have the following advantages. The applicator according to the present application includes a first housing and an insertion needle assembly, and the needle insertion assembly is disposed inside the first housing and is configured to carry a sensor assembly including the sensor. The needle insertion assembly includes a needle holder and a second housing. The needle holder is configured to fix the implant needle. The distal end of the second housing is provided with a first mounting seat for releasably accommodating the needle holder, and the proximal end of the second housing is configured to detachably connect with the sensor assembly. The first mounting seat is connected to a first elastic member arranged along the axial direction of the first housing. The distal end of the first elastic member is connected to the needle holder, and the proximal end of the first elastic member is connected to the proximal end of the first mounting seat. The first mounting seat includes a plurality of elastic claws, between which the first accommodation space for accommodating the needle holder and the first elastic member can be formed. The first housing includes a clasping member and a pressing portion. The clasping member is provided inside the first housing and arranged along the axial direction of the first housing. Before the needle insertion assembly is moved to a position close to the proximal end of the first housing, the clasping member can be sleeved on the elastic claws to clasp the elastic claws tightly. After the pressing portion is triggered, the needle insertion assembly is moved toward the proximal end of the first housing to percutaneously implant the sensor under the skin of the target subject until the clasping member can be separated from the elastic claws, so that the distal ends of the elastic claws can be separated from the needle holder, and then the first elastic member instantly causes the needle holder to automatically move toward the distal end of the first housing. Therefore, by triggering the pressing portion, the needle insertion assembly can be moved from a position away from the proximal end of the first housing to a position close to the proximal end of the first housing, so that the sensor assembly carrying the sensor can be transported to the skin surface of the target subject, and the sensor can be percutaneously implanted under the skin of the target subject to detect parameters such as blood glucose concentration in the blood of the target subject. The needle holder is releasably accommodated in the first mounting seat. Therefore, when the sensor is percutaneously implanted under the skin of the target subject, the clasping member can be separated from the elastic claws, so that the distal ends of the elastic claws can be separated from the needle holder, and then the needle holder can be instantly caused to automatically move toward the distal end of the first housing under the action of the resilience force of the first elastic member, so that the implant needle can instantly rebound together with the needle holder into the first housing, effectively avoiding accidental injury to the operator or other personnel by the sharp implant needle. In conclusion, the applicator according to the present application is not only simple in structure and low in cost, but also easier to operate. Since the medical device according to the present application includes the applicator described above, it has all the advantages of the medical device described above, so it will not be described in detail.

In addition, in the description of this specification, the reference terms "one/an embodiment", "some embodiments", "example", "specific example", or "some examples" etc. mean that the specific features, structures, materials or characteristics described in the embodiment(s) or example(s) are included in several embodiments or examples of the present application. In this specification, the exemplary expressions of the above terms do not necessarily refer to the same embodiment(s) or example(s). Furthermore, the specific features, structures, materials, or characteristics described may be combined in any suitable manner in any one or more embodiments or examples. Furthermore, those skilled in the art may combine and associate different embodiments or examples and features in different embodiments or examples described in this specification without mutual contradiction.

## Claims

1. An applicator for percutaneous implantation of a sensor, comprising a first housing (100) and a needle insertion assembly, wherein the needle insertion assembly is arranged inside the first housing (100) and is configured to carry a sensor assembly (700) comprising the sensor (710);
wherein the needle insertion assembly comprises a needle holder (300) and a second housing (400), wherein the needle holder (300) is configured to fix an implant needle (310), a first mounting seat (410) configured to releasably accommodate the needle holder (300) is provided at a distal end of the second housing (400), and a proximal end of the second housing (400) is configured to be detachably connected to the sensor assembly (700);
wherein the first mounting seat (410) is connected to a first elastic member (500) arranged along an axial direction of the first housing (100), a distal end of the first elastic member (500) is connected to the needle holder (300), and a proximal end of the first elastic member (500) is connected to a proximal end of the first mounting seat (410);
wherein the first mounting seat (410) comprises a plurality of elastic claws (412), between which a first accommodation space (413) configured to accommodate the needle holder (300) and the first elastic member (500) is formed;
wherein the first housing (100) comprises a clasping member (120) and a pressing portion, wherein the clasping member (120) is arranged inside the first housing (100) along the axial direction thereof, wherein before the needle insertion assembly is moved to a position close to a proximal end of the first housing (100), the clasping member (120) is sleeved over the elastic claws (412) to clasp the elastic claws (412); after the pressing portion is triggered, the needle insertion assembly is moved toward the proximal end of the first housing (100) to percutaneously implant the sensor (710) under a skin of a target subject until the clasping member (120) is separated from the elastic claws (412), so that distal ends of the elastic claws (412) can be separated from the needle holder (300), and then the first elastic member (500) instantly causes the needle holder (300) to automatically move toward a distal end of the first housing (100), wherein
the needle holder (300) comprises a fixing seat (320) and a plurality of guide pillars (330) extending along an axial direction of the fixing seat (320) toward the proximal end of the first housing (100), wherein a second accommodation space (340) configured to accommodate the first elastic member (500) is formed between the fixing seat (320) and the guide pillars (330), an opening is defined at a proximal end of the fixing seat (320), a connecting portion (321) is provided inside the fixing seat (320) along an axial direction thereof, a plurality of first clamping portions (311) are provided at a distal end of the implant needle (310), and first clamping slots (3211) matched with the first clamping portions (311) are provided on the connecting portion (321).

2. The applicator according to claim 1, wherein before the needle insertion assembly is moved to the position close to the proximal end of the first housing (100), the first elastic member (500) is in a compressed state so that the needle holder (300) is accommodated in the first mounting seat (410); wherein after the needle insertion assembly is moved to the position close to the proximal end of the first housing (100), under an action of the first elastic member (500), the needle holder (300) can be released from the first mounting seat (410) and move toward the distal end of the first housing (100).

3. The applicator according to claim 2, wherein the first mounting seat (410) further comprises a base (411), wherein the plurality of elastic claws (412) extend along an axial direction of the base (411) toward the distal end of the first housing (100), and the first accommodation space (413) is formed between the base (411) and the elastic claws (412); wherein before the needle insertion assembly is moved to the position close to the proximal end of the first housing (100), the distal ends of the elastic claws (412) abut against a distal end of the needle holder (300) to fix the needle holder (300) in the first accommodation space (413); wherein after the needle insertion assembly is moved to the position close to the proximal end of the first housing (100), the distal ends of the elastic claws (412) are separated from the needle holder (300) under the action of the first elastic member (500), so that the needle holder (300) can be released from the first mounting seat (410) and move toward the distal end of the first housing (100).

4. The applicator according to claim 1, wherein a second elastic member (600) is provided inside the first housing (100) along an axial direction thereof, a distal end of the second elastic member (600) is connected to the distal end of the first housing (100), and a proximal end of the second elastic member (600) is connected to the second housing (400); wherein before the pressing portion (200) is triggered, the second elastic member (600) is in a compressed state; wherein after the pressing portion (200) is triggered, under an action of the second elastic member (600), the second housing (400) moves toward the position close to the proximal end of the first housing (100).

5. The applicator according to claim 1, wherein a plurality of abutment members (420) are provided at the distal end of the second housing (400), an abutment platform (130) matched with the abutment members (420) is provided on an inner wall of the first housing (100), and the abutment platform (130) is close to a position where the pressing portion (200) is located; wherein before the pressing portion (200) is triggered, the abutment members (420) abut against the abutment platform (130); wherein after the pressing portion (200) is triggered, the abutment members (420) can be separated from the abutment platform (130).

6. The applicator according to claim 1, wherein the second housing (400) is provided with a sliding slot (430) extending through the proximal and distal ends of the second housing (400) along the axial direction of the first housing (100), a slide rail (140) matched with the sliding slot (430) is provided on an inner wall of the first housing (100), and a stopper (141) is provided at a proximal end of the slide rail (140).

7. The applicator according to claim 1, wherein the pressing portion (200) comprises a main body (210) and first cantilevers (220) arranged on both sides of the main body (210), wherein the first cantilevers (220) extend outward along the main body (210), the first cantilevers (220) are connected to an inner wall of the first housing (100), a first mounting hole (150) corresponding to the main body (210) is defined on the first housing (100), the main body (210) is installed in the first mounting hole (150), the main body (210) can move back and forth in the first mounting hole (150), the first cantilevers (220) each are arranged in an arc shape, a second mounting hole (221) is defined on a side of each of the first cantilevers (220) away from the main body (210), and a mounting pillar (160) matched with the second mounting hole (221) is provided on the inner wall of the first housing (100).

8. The applicator according to claim 1, wherein an anti-slip structure is provided on an outer wall of the first housing (100); wherein the anti-slip structure comprises one or more of ridges (110), bumps, recesses or textures arranged on the first housing (100).

9. The applicator according to claim 1, wherein a plurality of second cantilevers (312) are provided at the distal end of the implant needle (310), the second cantilevers (312) extend outward along an axial direction of the implant needle (310), and the first clamping portions (311) are provided on distal ends of the second cantilevers (312).

10. A medical device, comprising a sensor assembly (700) and the applicator according to any one of claims 1 to 9, wherein the sensor assembly (700) comprises a sensor (710) and a transmitter (720) that are connected with each other, and when the sensor (710) is percutaneously implanted under the skin of the target subject, the transmitter (720) can receive a detection result from the sensor (710) and transmit the detection result.

11. The medical device according to claim 10, wherein the sensor assembly (700) further comprises a foundation (730), wherein the sensor (710) and the transmitter (720) are installed on the foundation (730), and the proximal end of the second housing (400) is provided with a second mounting seat (440) configured to releasably accommodate the foundation (730), and the foundation (730) is detachably connected to the second mounting seat (440).

12. The medical device according to claim 11, wherein a plurality of second clamping slots (731) are provided on an outer periphery of the foundation (730), and second clamping portions (441) matched with the second clamping slots (731) are provided on an inner wall of the second mounting seat (440).

13. The medical device according to claim 12, wherein a through hole (732) allowing the implant needle (310) to pass therethrough is defined on the foundation (730), a proximal end of the sensor (710) is provided with a pin (712) protruding out of the through hole (732), and a groove (313) configured to wrap the pin (712) is defined on the implant needle (310) along an axial direction thereof.

14. The medical device according to claim 11, wherein a proximal end of the foundation (730) is provided with a contact surface (750) extending outwardly along a circumferential direction of the foundation (730), the contact surface (750) is configured to contact the skin of the target subject, and an adhesive is provided on the contact surface (750).

15. The medical device according to claim 10, wherein a through hole (732) allowing the implant needle (310) of the applicator to pass therethrough is defined on the sensor assembly (700), and an elastic contact switch (760) is provided in the through hole (732), when the needle holder (300) is accommodated in the first mounting seat (410), the elastic contact switch (760) is in a non-conducting state, and when the needle holder (300) is released from the first mounting seat (410) and moves to a position close to the distal end of the first housing (100), the elastic contact switch (760) is in a conducting state.

## Patentansprüche

1. Applikator zur perkutanen Implantation eines Sensors, umfassend ein erstes Gehäuse (100) und eine Nadeleinführungsanordnung, wobei die Nadeleinführungsanordnung innerhalb des ersten Gehäuses (100) angeordnet und ausgebildet ist, eine Sensoranordnung (700) zu tragen, die den Sensor (710) umfasst;
wobei die Nadeleinführungsanordnung einen Nadelhalter (300) und ein zweites Gehäuse (400) umfasst, wobei der Nadelhalter (300) ausgebildet ist, eine Implantationsnadel (310) zu fixieren, ein erster Montagesitz (410), der ausgebildet ist, den Nadelhalter (300) lösbar aufzunehmen, an einem distalen Ende des zweiten Gehäuses (400) vorgesehen ist und ein proximales Ende des zweiten Gehäuses (400) ausgebildet ist, lösbar mit der Sensoranordnung (700) verbunden zu werden;
wobei der erste Montagesitz (410) mit einem ersten elastischen Element (500) verbunden ist, das entlang einer axialen Richtung des ersten Gehäuses (100) angeordnet ist, ein distales Ende des ersten elastischen Elements (500) mit dem Nadelhalter (300) verbunden ist und ein proximales Ende des ersten elastischen Elements (500) mit einem proximalen Ende des ersten Montagesitzes (410) verbunden ist;
wobei der erste Montagesitz (410) eine Vielzahl elastischer Klauen (412) umfasst, zwischen denen ein erster Aufnahmeraum (413) ausgebildet ist, der ausgebildet ist, den Nadelhalter (300) und das erste elastische Element (500) aufzunehmen;
wobei das erste Gehäuse (100) ein Klammerelement (120) und einen Druckabschnitt umfasst, wobei das Klammerelement (120) innerhalb des ersten Gehäuses (100) entlang dessen axialer Richtung angeordnet ist, wobei, bevor die Nadeleinführungsanordnung zu einer Position nahe einem proximalen Ende des ersten Gehäuses (100) bewegt wird, das Klammerelement (120) über die elastischen Klauen (412) gestülpt ist, um die elastischen Klauen (412) zu verklammern; nachdem der Druckabschnitt ausgelöst worden ist, wird die Nadeleinführungsanordnung in Richtung des proximalen Endes des ersten Gehäuses (100) bewegt, um den Sensor (710) perkutan unter der Haut eines Zielsubjekts zu implantieren, bis das Klammerelement (120) von den elastischen Klauen (412) getrennt ist, sodass distale Enden der elastischen Klauen (412) von dem Nadelhalter (300) getrennt werden können, und dann bewirkt das erste elastische Element (500) sofort, dass sich der Nadelhalter (300) automatisch in Richtung eines distalen Endes des ersten Gehäuses (100) bewegt, wobei:
der Nadelhalter (300) einen Befestigungssitz (320) und eine Vielzahl von Führungssäulen (330) umfasst, die sich entlang einer axialen Richtung des Befestigungssitzes (320) in Richtung des proximalen Endes des ersten Gehäuses (100) erstrecken, wobei ein zweiter Aufnahmeraum (340), der ausgebildet ist, das erste elastische Element (500) aufzunehmen, zwischen dem Befestigungssitz (320) und den Führungssäulen (330) ausgebildet ist, eine Öffnung an einem proximalen Ende des Befestigungssitzes (320) definiert ist, ein Verbindungsabschnitt (321) innerhalb des Befestigungssitzes (320) entlang einer axialen Richtung desselben vorgesehen ist, eine Vielzahl erster Klemmabschnitte (311) an einem distalen Ende der Implantationsnadel (310) vorgesehen sind und erste Klemmschlitze (3211), die zu den ersten Klemmabschnitten (311) passen, an dem Verbindungsabschnitt (321) vorgesehen sind.

2. Applikator nach Anspruch 1, wobei, bevor die Nadeleinführungsanordnung zu der Position nahe dem proximalen Ende des ersten Gehäuses (100) bewegt wird, das erste elastische Element (500) in einem komprimierten Zustand ist, sodass der Nadelhalter (300) in dem ersten Montagesitz (410) aufgenommen ist; wobei, nachdem die Nadeleinführungsanordnung zu der Position nahe dem proximalen Ende des ersten Gehäuses (100) bewegt worden ist, unter Einwirkung des ersten elastischen Elements (500) der Nadelhalter (300) von dem ersten Montagesitz (410) freigegeben werden und sich in Richtung des distalen Endes des ersten Gehäuses (100) bewegen kann.

3. Applikator nach Anspruch 2, wobei der erste Montagesitz (410) ferner eine Basis (411) umfasst, wobei sich die Vielzahl elastischer Klauen (412) entlang einer axialen Richtung der Basis (411) in Richtung des distalen Endes des ersten Gehäuses (100) erstreckt und der erste Aufnahmeraum (413) zwischen der Basis (411) und den elastischen Klauen (412) ausgebildet ist; wobei, bevor die Nadeleinführungsanordnung zu der Position nahe dem proximalen Ende des ersten Gehäuses (100) bewegt wird, die distalen Enden der elastischen Klauen (412) an einem distalen Ende des Nadelhalters (300) anliegen, um den Nadelhalter (300) in dem ersten Aufnahmeraum (413) zu fixieren; wobei, nachdem die Nadeleinführungsanordnung zu der Position nahe dem proximalen Ende des ersten Gehäuses (100) bewegt worden ist, die distalen Enden der elastischen Klauen (412) unter der Einwirkung des ersten elastischen Elements (500) von dem Nadelhalter (300) getrennt werden, sodass der Nadelhalter (300) von dem ersten Montagesitz (410) freigegeben werden und sich in Richtung des distalen Endes des ersten Gehäuses (100) bewegen kann.

4. Applikator nach Anspruch 1, wobei ein zweites elastisches Element (600) innerhalb des ersten Gehäuses (100) entlang einer axialen Richtung desselben vorgesehen ist, ein distales Ende des zweiten elastischen Elements (600) mit dem distalen Ende des ersten Gehäuses (100) verbunden ist und ein proximales Ende des zweiten elastischen Elements (600) mit dem zweiten Gehäuse (400) verbunden ist; wobei, bevor der Druckabschnitt (200) ausgelöst wird, das zweite elastische Element (600) in einem komprimierten Zustand ist; wobei, nachdem der Druckabschnitt (200) ausgelöst worden ist, sich das zweite Gehäuse (400) unter Einwirkung des zweiten elastischen Elements (600) zu der Position nahe dem proximalen Ende des ersten Gehäuses (100) bewegt.

5. Applikator nach Anspruch 1, wobei eine Vielzahl von Anlageelementen (420) an dem distalen Ende des zweiten Gehäuses (400) vorgesehen ist, eine Anlageplattform (130), die zu den Anlageelementen (420) passt, an einer Innenwand des ersten Gehäuses (100) vorgesehen ist und sich die Anlageplattform (130) nahe einer Position befindet, an der sich der Druckabschnitt (200) befindet; wobei, bevor der Druckabschnitt (200) ausgelöst wird, die Anlageelemente (420) an der Anlageplattform (130) anliegen; wobei, nachdem der Druckabschnitt (200) ausgelöst worden ist, die Anlageelemente (420) von der Anlageplattform (130) getrennt werden können.

6. Applikator nach Anspruch 1, wobei das zweite Gehäuse (400) mit einem Gleitschlitz (430) versehen ist, der sich von dem proximalen und zu dem distalen Ende des zweiten Gehäuses (400) entlang der axialen Richtung des ersten Gehäuses (100) erstreckt, eine Gleitschiene (140), die zu dem Gleitschlitz (430) passt, an einer Innenwand des ersten Gehäuses (100) vorgesehen ist und ein Anschlag (141) an einem proximalen Ende der Gleitschiene (140) vorgesehen ist.

7. Applikator nach Anspruch 1, wobei der Druckabschnitt (200) einen Hauptkörper (210) und erste Federarme (220) umfasst, die auf beiden Seiten des Hauptkörpers (210) angeordnet sind, wobei sich die ersten Federarme (220) entlang des Hauptkörpers (210) nach außen erstrecken, die ersten Federarme (220) mit einer Innenwand des ersten Gehäuses (100) verbunden sind, eine erste Montageöffnung (150), die dem Hauptkörper (210) entspricht, an dem ersten Gehäuse (100) definiert ist, der Hauptkörper (210) in der ersten Montageöffnung (150) installiert ist, sich der Hauptkörper (210) in der ersten Montageöffnung (150) hin und her bewegen kann, die ersten Federarme (220) jeweils bogenförmig angeordnet sind, eine zweite Montageöffnung (221) an einer Seite jedes der ersten Federarme (220), die von dem Hauptkörper (210) abgewandt ist, definiert ist und ein Montagezapfen (160), der zu der zweiten Montageöffnung (221) passt, an der Innenwand des ersten Gehäuses (100) vorgesehen ist.

8. Applikator nach Anspruch 1, wobei eine Rutschschutzstruktur an einer Außenwand des ersten Gehäuses (100) vorgesehen ist; wobei die Rutschschutzstruktur eines oder mehrere von Rippen (110), Erhebungen, Vertiefungen oder Texturen umfasst, die an dem ersten Gehäuse (100) angeordnet sind.

9. Applikator nach Anspruch 1, wobei eine Vielzahl zweiter Federarme (312) an dem distalen Ende der Implantationsnadel (310) vorgesehen ist, sich die zweiten Federarme (312) entlang einer axialen Richtung der Implantationsnadel (310) nach außen erstrecken und die ersten Klemmabschnitte (311) an distalen Enden der zweiten Federarme (312) vorgesehen sind.

10. Medizinische Vorrichtung, umfassend eine Sensoranordnung (700) und den Applikator nach einem der Ansprüche 1 bis 9, wobei die Sensoranordnung (700) einen Sensor (710) und einen Sender (720) umfasst, die miteinander verbunden sind, und wenn der Sensor (710) perkutan unter die Haut des Zielsubjekts implantiert wird, der Sender (720) ein Erfassungsergebnis von dem Sensor (710) empfangen und das Erfassungsergebnis übertragen kann.

11. Medizinische Vorrichtung nach Anspruch 10, wobei die Sensoranordnung (700) ferner eine Grundplatte (730) umfasst, wobei der Sensor (710) und der Sender (720) auf der Grundplatte (730) installiert sind und das proximale Ende des zweiten Gehäuses (400) mit einem zweiten Montagesitz (440) versehen ist, der ausgebildet ist, die Grundplatte (730) lösbar aufzunehmen, und die Grundplatte (730) lösbar mit dem zweiten Montagesitz (440) verbunden ist.

12. Medizinische Vorrichtung nach Anspruch 11, wobei eine Vielzahl zweiter Klemmschlitze (731) an einem Außenumfang der Grundplatte (730) vorgesehen ist und zweite Klemmabschnitte (441), die zu den zweiten Klemmschlitzen (731) passen, an einer Innenwand des zweiten Montagesitzes (440) vorgesehen sind.

13. Medizinische Vorrichtung nach Anspruch 12, wobei ein Durchgangsloch (732), durch das die Implantationsnadel (310) hindurchtreten kann, an der Grundplatte (730) definiert ist, ein proximales Ende des Sensors (710) mit einem Stift (712) versehen ist, der aus dem Durchgangsloch (732) hervorsteht, und eine Nut (313), die ausgebildet ist, den Stift (712) zu umschließen, an der Implantationsnadel (310) entlang einer axialen Richtung derselben definiert ist.

14. Medizinische Vorrichtung nach Anspruch 11, wobei ein proximales Ende der Grundplatte (730) mit einer Kontaktfläche (750) versehen ist, die sich entlang einer Umfangsrichtung der Grundplatte (730) nach außen erstreckt, die Kontaktfläche (750) ausgebildet ist, die Haut des Zielsubjekts zu berühren, und ein Klebstoff auf der Kontaktfläche (750) vorgesehen ist.

15. Medizinische Vorrichtung nach Anspruch 10, wobei ein Durchgangsloch (732), durch das die Implantationsnadel (310) des Applikators hindurchtreten kann, an der Sensoranordnung (700) definiert ist und ein elastischer Kontaktschalter (760) in dem Durchgangsloch (732) vorgesehen ist, wobei, wenn der Nadelhalter (300) in dem ersten Montagesitz (410) aufgenommen ist, der elastische Kontaktschalter (760) in einem nichtleitenden Zustand ist, und wenn der Nadelhalter (300) von dem ersten Montagesitz (410) freigegeben wird und sich zu einer Position nahe dem distalen Ende des ersten Gehäuses (100) bewegt, der elastische Kontaktschalter (760) in einem leitenden Zustand ist.

## Revendications

1. Applicateur pour l'implantation percutanée d'un capteur, comprenant un premier boîtier (100) et un ensemble d'insertion d'aiguille, dans lequel l'ensemble d'insertion d'aiguille est agencé à l'intérieur du premier boîtier (100) et est configuré pour porter un ensemble capteur (700) comprenant le capteur (710) ;
dans lequel l'ensemble d'insertion d'aiguille comprend un porte-aiguille (300) et un second boîtier (400), dans lequel le porte-aiguille (300) est configuré pour fixer une aiguille d'implantation (310), un premier siège de montage (410) configuré pour recevoir de manière libérable le porte-aiguille (300) est prévu à une extrémité distale du second boîtier (400), et une extrémité proximale du second boîtier (400) est configurée pour être connectée de manière amovible à l'ensemble capteur (700) ;
dans lequel le premier siège de montage (410) est relié à un premier élément élastique (500) agencé le long d'une direction axiale du premier boîtier (100), une extrémité distale du premier élément élastique (500) est reliée au porte-aiguille (300), et une extrémité proximale du premier élément élastique (500) est reliée à une extrémité proximale du premier siège de montage (410) ;
dans lequel le premier siège de montage (410) comprend une pluralité de griffes élastiques (412), entre lesquelles est formé un premier espace de réception (413) configuré pour recevoir le porte-aiguille (300) et le premier élément élastique (500) ;
dans lequel le premier boîtier (100) comprend un élément de serrage (120) et une partie de pression, dans lequel l'élément de serrage (120) est agencé à l'intérieur du premier boîtier (100) le long de la direction axiale de celui-ci, dans lequel, avant que l'ensemble d'insertion d'aiguille ne soit déplacé vers une position proche d'une extrémité proximale du premier boîtier (100), l'élément de serrage (120) est monté autour des griffes élastiques (412) pour serrer les griffes élastiques (412) ; après que la partie de pression a été déclenchée, l'ensemble d'insertion d'aiguille est déplacé vers l'extrémité proximale du premier boîtier (100) pour implanter percutanément le capteur (710) sous la peau d'un sujet cible jusqu'à ce que l'élément de serrage (120) soit séparé des griffes élastiques (412), de sorte que des extrémités distales des griffes élastiques (412) puissent être séparées du porte-aiguille (300), et ensuite le premier élément élastique (500) amène instantanément le porte-aiguille (300) à se déplacer automatiquement vers une extrémité distale du premier boîtier (100), dans lequel :
le porte-aiguille (300) comprend un siège de fixation (320) et une pluralité de colonnes de guidage (330) s'étendant le long d'une direction axiale du siège de fixation (320) vers l'extrémité proximale du premier boîtier (100), dans lequel un second espace de réception (340) configuré pour recevoir le premier élément élastique (500) est formé entre le siège de fixation (320) et les colonnes de guidage (330), une ouverture est définie à une extrémité proximale du siège de fixation (320), une partie de liaison (321) est prévue à l'intérieur du siège de fixation (320) le long d'une direction axiale de celui-ci, une pluralité de premières parties de serrage (311) sont prévues à une extrémité distale de l'aiguille d'implantation (310), et des premières fentes de serrage (3211) coopérant avec les premières parties de serrage (311) sont prévues sur la partie de liaison (321).

2. Applicateur selon la revendication 1, dans lequel, avant que l'ensemble d'insertion d'aiguille ne soit déplacé vers la position proche de l'extrémité proximale du premier boîtier (100), le premier élément élastique (500) est dans un état comprimé de sorte que le porte-aiguille (300) est reçu dans le premier siège de montage (410) ; dans lequel, après que l'ensemble d'insertion d'aiguille a été déplacé vers la position proche de l'extrémité proximale du premier boîtier (100), sous l'action du premier élément élastique (500), le porte-aiguille (300) peut être libéré du premier siège de montage (410) et se déplacer vers l'extrémité distale du premier boîtier (100).

3. Applicateur selon la revendication 2, dans lequel le premier siège de montage (410) comprend en outre une base (411), dans lequel la pluralité de griffes élastiques (412) s'étendent le long d'une direction axiale de la base (411) vers l'extrémité distale du premier boîtier (100), et le premier espace de réception (413) est formé entre la base (411) et les griffes élastiques (412) ; dans lequel, avant que l'ensemble d'insertion d'aiguille ne soit déplacé vers la position proche de l'extrémité proximale du premier boîtier (100), les extrémités distales des griffes élastiques (412) viennent en butée contre une extrémité distale du porte-aiguille (300) pour fixer le porte-aiguille (300) dans le premier espace de réception (413) ; dans lequel, après que l'ensemble d'insertion d'aiguille a été déplacé vers la position proche de l'extrémité proximale du premier boîtier (100), les extrémités distales des griffes élastiques (412) sont séparées du porte-aiguille (300) sous l'action du premier élément élastique (500), de sorte que le porte-aiguille (300) puisse être libéré du premier siège de montage (410) et se déplacer vers l'extrémité distale du premier boîtier (100).

4. Applicateur selon la revendication 1, dans lequel un second élément élastique (600) est prévu à l'intérieur du premier boîtier (100) le long d'une direction axiale de celui-ci, une extrémité distale du second élément élastique (600) est reliée à l'extrémité distale du premier boîtier (100), et une extrémité proximale du second élément élastique (600) est reliée au second boîtier (400) ; dans lequel, avant que la partie de pression (200) ne soit déclenchée, le second élément élastique (600) est dans un état comprimé ; dans lequel, après que la partie de pression (200) a été déclenchée, sous l'action du second élément élastique (600), le second boîtier (400) se déplace vers la position proche de l'extrémité proximale du premier boîtier (100).

5. Applicateur selon la revendication 1, dans lequel une pluralité d'éléments de butée (420) sont prévus à l'extrémité distale du second boîtier (400), une plateforme de butée (130) coopérant avec les éléments de butée (420) est prévue sur une paroi intérieure du premier boîtier (100), et la plateforme de butée (130) est proche d'une position où se trouve la partie de pression (200) ; dans lequel, avant que la partie de pression (200) ne soit déclenchée, les éléments de butée (420) viennent en butée contre la plateforme de butée (130) ; dans lequel, après que la partie de pression (200) a été déclenchée, les éléments de butée (420) peuvent être séparés de la plateforme de butée (130).

6. Applicateur selon la revendication 1, dans lequel le second boîtier (400) est pourvu d'une fente de coulissement (430) s'étendant de l'extrémité proximale à l'extrémité distale du second boîtier (400) le long de la direction axiale du premier boîtier (100), un rail de guidage (140) coopérant avec la fente de coulissement (430) est prévu sur une paroi intérieure du premier boîtier (100), et une butée (141) est prévue à une extrémité proximale du rail de guidage (140).

7. Applicateur selon la revendication 1, dans lequel la partie de pression (200) comprend un corps principal (210) et des premiers bras en porte-à-faux (220) agencés sur les deux côtés du corps principal (210), dans lequel les premiers bras en porte-à-faux (220) s'étendent vers l'extérieur le long du corps principal (210), les premiers bras en porte-à-faux (220) sont reliés à une paroi intérieure du premier boîtier (100), un premier orifice de montage (150) correspondant au corps principal (210) est défini sur le premier boîtier (100), le corps principal (210) est installé dans le premier orifice de montage (150), le corps principal (210) peut se déplacer d'un mouvement de va-et-vient dans le premier orifice de montage (150), les premiers bras en porte-à-faux (220) sont chacun agencés selon une forme arquée, un second orifice de montage (221) est défini sur un côté de chacun des premiers bras en porte-à-faux (220) éloigné du corps principal (210), et un tourillon de montage (160) coopérant avec le second orifice de montage (221) est prévu sur la paroi intérieure du premier boîtier (100).

8. Applicateur selon la revendication 1, dans lequel une structure antidérapante est prévue sur une paroi extérieure du premier boîtier (100) ; dans lequel la structure antidérapante comprend une ou plusieurs nervures (110), bosses, cavités ou textures agencées sur le premier boîtier (100).

9. Applicateur selon la revendication 1, dans lequel une pluralité de seconds bras en porte-à-faux (312) sont prévus à l'extrémité distale de l'aiguille d'implantation (310), les seconds bras en porte-à-faux (312) s'étendent vers l'extérieur le long d'une direction axiale de l'aiguille d'implantation (310), et les premières parties de serrage (311) sont prévues sur des extrémités distales des seconds bras en porte-à-faux (312).

10. Dispositif médical, comprenant un ensemble capteur (700) et l'applicateur selon l'une quelconque des revendications 1 à 9, dans lequel l'ensemble capteur (700) comprend un capteur (710) et un émetteur (720) qui sont reliés l'un à l'autre, et lorsque le capteur (710) est implanté percutanément sous la peau du sujet cible, l'émetteur (720) peut recevoir un résultat de détection provenant du capteur (710) et transmettre le résultat de détection.

11. Dispositif médical selon la revendication 10, dans lequel l'ensemble capteur (700) comprend en outre un socle (730), dans lequel le capteur (710) et l'émetteur (720) sont installés sur le socle (730), et l'extrémité proximale du second boîtier (400) est pourvue d'un second siège de montage (440) configuré pour recevoir de manière libérable le socle (730), et le socle (730) est relié de manière amovible au second siège de montage (440).

12. Dispositif médical selon la revendication 11, dans lequel une pluralité de secondes fentes de serrage (731) sont prévues sur une périphérie extérieure du socle (730), et des secondes parties de serrage (441) coopérant avec les secondes fentes de serrage (731) sont prévues sur une paroi intérieure du second siège de montage (440).

13. Dispositif médical selon la revendication 12, dans lequel un trou traversant (732) permettant à l'aiguille d'implantation (310) de le traverser est défini sur le socle (730), une extrémité proximale du capteur (710) est pourvue d'une broche (712) faisant saillie hors du trou traversant (732), et une rainure (313) configurée pour envelopper la broche (712) est définie sur l'aiguille d'implantation (310) le long d'une direction axiale de celle-ci.

14. Dispositif médical selon la revendication 11, dans lequel une extrémité proximale du socle (730) est pourvue d'une surface de contact (750) s'étendant vers l'extérieur le long d'une direction circonférentielle du socle (730), la surface de contact (750) est configurée pour entrer en contact avec la peau du sujet cible, et un adhésif est prévu sur la surface de contact (750).

15. Dispositif médical selon la revendication 10, dans lequel un trou traversant (732) permettant à l'aiguille d'implantation (310) de l'applicateur de le traverser est défini sur l'ensemble capteur (700), et un commutateur de contact élastique (760) est prévu dans le trou traversant (732), dans lequel, lorsque le porte-aiguille (300) est reçu dans le premier siège de montage (410), le commutateur de contact élastique (760) est dans un état non conducteur, et lorsque le porte-aiguille (300) est libéré du premier siège de montage (410) et se déplace vers une position proche de l'extrémité distale du premier boîtier (100), le commutateur de contact élastique (760) est dans un état conducteur.
